# EUROPEAN PATENT APPLICATION

(11) **EP 4 151 919 A1**
(43) Date of publication of application: **22.03.2023**
(21) Application number: 22195825.9
(22) Date of filing: 15.09.2022
(51) Int. Cl.: F24F 8/30

(54) **AEROSOL PURIFIER FOR HVAC SYSTEM**

(30) Priority: 15.09.2021 IN 202131041607; 29.11.2021 US 202117456797
(71) Applicant: Trane International Inc., Davidson, NC 28036 (US)
(72) Inventor: VARGHESE, Zubin, North Carolina, 28036 (US); BALEGUNDI, Rohith L, North Carolina, 28036 (US); PANDIT, Yogesh, North Carolina, 28036 (US); VEGEESHWARA, Sandeep, North Carolina, 28036 (US); HASANEEN, Rasha, North Carolina, 28036 (US); GOUKER, Joel Patrick, North Carolina, 28036 (US); BERGMAN, Jennie, North Carolina, 28036 (US); COSBY, Ronald, North Carolina, 28036 (US)
(74) Representative: Haseltine Lake Kempner LLP

(57) **Abstract**

An air purifying device, system and method for cleaning and infecting a conditioned space or zone. The air purifying system may include a heating, ventilation and air conditioning (HVAC) system with HVAC equipment configured to provide conditioned air to a conditioned space, where the HVAC system also includes a condensate trap configured to collect condensate produced during operation. The air purifying system also including an air purifying device configured to energize the condensate from the condensate trap to generate an aerosol including negative ions, which are combined the conditioned air provided by the HVAC system to the conditioned space.

## Description

### TECHNOLOGICAL FIELD

The present disclosure relates generally to an aerosol purifier which may be used with a heating, ventilation, and/or air conditioning (HVAC) systems for supplying an aerosol with negative ions to a conditioned space, ensuring good air quality and purifying the space.

### BACKGROUND

Climate control systems, such as heating, ventilation, and/or air conditioning (HVAC) systems are used in residential, industrial, and commercial buildings to heat, cool or otherwise condition spaces by providing conditioned air. These systems often include various components or devices for cleaning air circulated through the device. However, these existing solutions have various limitations, particularly for smaller contaminants and surfaces within the room.

Solid particle air filters such as HEPA filters clean recirculated air and provide acceptable cleaning for many circumstances, however these filters are often limited in terms of the size particles that may be filters. In addition, they generate static pressure losses within the HVAC system, which leads to energy losses. As a result, there is an inverse relationship between particle size/cleaning efficacy and energy efficiency. These filters are also passive elements and, thus, are limited to removing particles from the circulating air prior to entering a space. They are not capable of actively generating cleaning solutions that may be delivered by the HVAC system to the space.

Moreover, existing active cleaning technologies used in the HVAC systems are limited and suffers various drawbacks. For example, electrical field chargers typically use a corona discharge to create an electric field and ionize particles in the air. Given the high power levels and the varying types of contaminates within the air stream, these systems often generate undesired levels of ozone. Similarly, chemical cleaners often generate various cleaning solutions such as hydrogen peroxide, however, these solutions may have undesirable side effects, including the creation of ozone.

Outside the HVAC industry, technologies exist that are capable of generating ions using water. These technologies may utilize water shearing or the Lenard effect, in which electrical charges are generated when kinetic forces break up water into droplets. These droplets may collide with each other to generate ions, which may be used for cleaning in some instances. However, to date, none of these systems utilizes an air purifying device coupled to a HVAC device, particularly not one that utilizes discharge condensate from the HVAC system to generate the cleaning aerosol. Thus, the system described herein has various advantages over the existing technologies.

### BRIEF SUMMARY

Example implementations of the present disclosure provide an air purifying system and associated methods for use with an HVAC system that generates cleaner air for a conditioned space. These air purifying devices, in some examples, use condensate generated by various components of the HVAC system to generate an aerosol to clean the air. The condensate is routed to the aerosol generator, which energizes the water to create an aerosol containing negative ions. This aerosol mixes with the conditioned air and is routed to a conditioned space (and potentially an outside environment), often cleaning both the air and surfaces within the space. Additional control circuitry is also used in some instances to provide enhanced control and operation of the device and system.

The present disclosure thus includes, without limitation, the following example implementations.

Some example implementations provide an air purifying device for a heating, ventilation and air conditioning (HVAC) system operable to provide conditioned air to a conditioned space, the HVAC system including a condensate trap configured to collect condensate produced during operation, the air purifying device comprising: an aerosol generator; and a conduit operably coupleable to and between the condensate trap and the aerosol generator, the conduit configured to deliver the condensate from the condensate trap to the aerosol generator that is configured to energize the condensate to generate an aerosol including negative ions combinable with the conditioned air provided by the HVAC system to the conditioned space.

Some example implementations provide an air purifying system comprising: a heating, ventilation and air conditioning (HVAC) system with HVAC equipment configured to provide conditioned air to a conditioned space, the HVAC system including a condensate trap configured to collect condensate produced during operation; and an air purifying device configured to energize the condensate from the condensate trap to generate an aerosol including negative ions, the aerosol being combinable with the conditioned air provided by the HVAC system to the conditioned space.

Some example implementations provide a method of disinfecting a conditioned space to which heating, ventilation, and air conditioning (HVAC) equipment of an HVAC system is configured to provide conditioned air, the HVAC system including a condensate trap configured to collect condensate produced during operation, the method comprising: delivering the condensate from the condensate trap to an aerosol generator; energizing the condensate at the aerosol generator to generate an aerosol including negative ions; combining the aerosol including the negative ions with the conditioned air provided by the HVAC system; and delivering the combined air and aerosol to the conditioned space.

These and other features, aspects, and advantages of the present disclosure will be apparent from a reading of the following detailed description together with the accompanying figures, which are briefly described below. The present disclosure includes any combination of two, three, four or more features or elements set forth in this disclosure, regardless of whether such features or elements are expressly combined or otherwise recited in a specific example implementation described herein. This disclosure is intended to be read holistically such that any separable features or elements of the disclosure, in any of its aspects and example implementations, should be viewed as combinable unless the context of the disclosure clearly dictates otherwise.

It will therefore be appreciated that this Brief Summary is provided merely for purposes of summarizing some example implementations so as to provide a basic understanding of some aspects of the disclosure. Accordingly, it will be appreciated that the above described example implementations are merely examples and should not be construed to narrow the scope or spirit of the disclosure in any way. Other example implementations, aspects and advantages will become apparent from the following detailed description taken in conjunction with the accompanying figures which illustrate, by way of example, the principles of some described example implementations.

### BRIEF DESCRIPTION OF THE FIGURE(S)

Having thus described example implementations of the disclosure in general terms, reference will now be made to the accompanying figures, which are not necessarily drawn to scale, and wherein:
FIG. 1A is a block diagram of an air purifying system coupled to a heating, ventilation, and air conditioning (HVAC) system according to some example implementations of the present disclosure;
FIG. 1B is a block diagram of an air purifying system with an aromatic dispenser coupled to a heating, ventilation, and air conditioning (HVAC) system according to some example implementations of the present disclosure;
FIG. 2A is an illustration of an air handling unit with an air purifying device, according to some example implementations;
FIG. 2B is another illustration of an air handling unit with an air purifying device, according to some example implementations;
FIG. 3 is an illustration of an air purifying device, according to some example implementations;
FIG. 4 is an illustration of a condensate trap, according to some example implementations;
FIG. 5A is an illustration of an aromatic dispenser, according to some example implementations;
FIG. 5B is an illustration of various components of an aromatic dispenser, according to some example implementations;
FIG. 6 is an illustration of various conditioned spaces that include air handing units and air purifying devices, according to some example implementations;
FIG. 7 is a diagram of a purifying device, according to some example implementations;
FIG. 8 is an illustration of a refrigerant circuit that may be used to form a disinfectant, according to some example implementations;
FIGS. 9A, 9B, 9C, 9D, 9E, 9F, 9G, 9H, and 91 are flowcharts illustrating various operations in a method of controlling an air purifying system, according to some example implementations;
FIGS. 10A, 10B, 10C, 10D, 10E, 10F, 10G, 10H, and 101 are flowcharts illustrating various operations in a method of controlling an air purifying system, according to some example implementations;
FIGS. 11A, 11B, 11C, 11D, 11E, 11F, and 11G are flowcharts illustrating various operations in a method of controlling an air purifying system, according to some example implementations;
FIGS. 12A, 12B, 12C, 12D, and 12E are flowcharts illustrating various operations in a method of controlling a purifying system, according to some example implementations; and
FIG. 13 illustrates control circuitry according to some example implementations.

### DETAILED DESCRIPTION

Some implementations of the present disclosure will now be described more fully hereinafter with reference to the accompanying figures, in which some, but not all implementations of the disclosure are shown. Indeed, various implementations of the disclosure may be embodied in many different forms and should not be construed as limited to the implementations set forth herein; rather, these example implementations are provided so that this disclosure will be thorough and complete, and will fully convey the scope of the disclosure to those skilled in the art. Like reference numerals refer to like elements throughout.

Unless specified otherwise or clear from context, references to first, second or the like should not be construed to imply a particular order. A feature described as being above another feature (unless specified otherwise or clear from context) may instead be below, and vice versa; and similarly, features described as being to the left of another feature may instead be to the right, and vice versa. Also, while reference may be made herein to quantitative measures, values, geometric relationships or the like, unless otherwise stated, any one or more if not all of these may be absolute or approximate to account for acceptable variations that may occur, such as those due to engineering tolerances or the like.

As used herein, unless specified otherwise or clear from context, the "or" of a set of operands is the "inclusive or" and thereby true if and only if one or more of the operands is true, as opposed to the "exclusive or" which is false when all of the operands are true. Thus, for example, "[A] or [B]" is true if [A] is true, or if [B] is true, or if both [A] and [B] are true. Further, the articles "a" and "an" mean "one or more," unless specified otherwise or clear from context to be directed to a singular form. Furthermore, it should be understood that unless otherwise specified, the terms "data," "content," "digital content," "information," "observation" and similar terms may be at times used interchangeably.

Example implementations of the present disclosure provide an air purifying device for use with an HVAC system and associated methods for controlling the components therein. The air purifying device may be coupled to a condensate producing component within the HVAC system where the condensate is collected by a condensate trap and routed to an aerosol generator. In some instances, the condensate is purified and/or disinfected prior to entering the aerosol generator. The aerosol generator energizes the condensate, creating an aerosol, which often is composed of water vapor. Negative ions are also created from the condensate as part of this process, and the aerosol with the negative ions are combined with conditioned air and routed to a conditioned space. The aerosol and the negative ions may clean the conditioned air as well as the conditioned space, and in some instances, they may also clean surfaces within the space, potentially prior to the space being occupied.

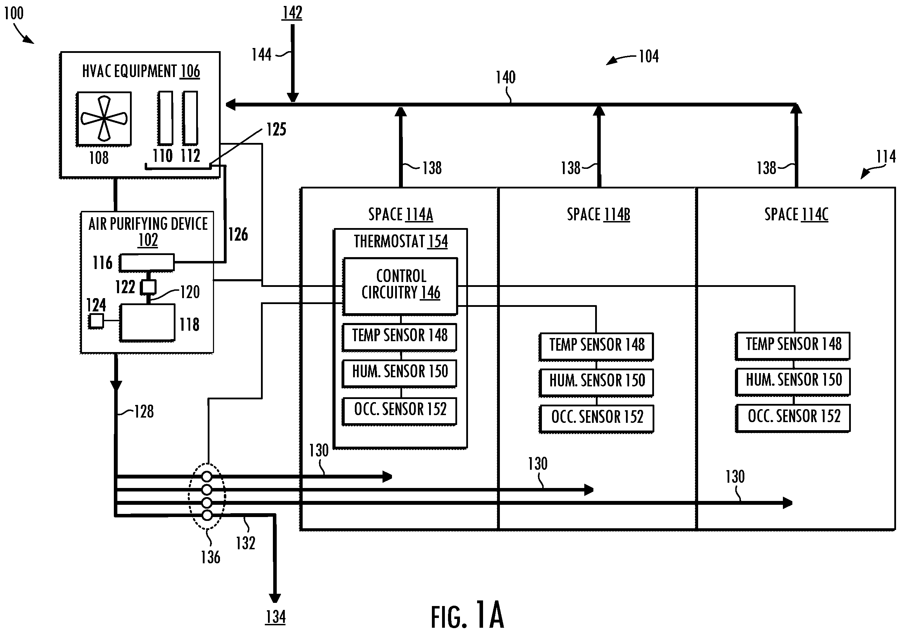

FIG. **1A** is a block diagram of the air purifying system **100** according to some example implementations of the present disclosure. This system includes an air purifying device **102** coupled to an HVAC system **104** that generally includes HVAC equipment **106** with a fan **108,** cooling element **110,** and/or heating elements **112.** The cooling element and/or heating element may comprise a heat exchanger coil. The fan **108** may be an indoor fan or blower configured to provide conditioned air to a conditioned space **114,** which in some examples may be divided into a plurality of spaces or zones **114A, 114B, 114C** (each of which may itself be a conditioned space). It will be appreciated that while three spaces are shown, any number of spaces or zones may be present. It will also be appreciated that references to the conditioned space may be equally applicable to one or more spaces, and/or zones each of which may be comprised of one or more spaces.

The air purifying device **102** may include a condensate trap **116,** an aerosol generator **118,** a conduit **120,** a condensate pump **122,** and a drive circuit **124.** The condensate trap collects condensate produced by a condensate producing component. Often this component is the cooling element **110,** particularly a cooling coil, but other components within the HVAC system may also produce condensate that may be collected by the condensate trap and used by the air purifying device. In some examples, a drain pan **125** is positioned under the condensate producing component and that pan is coupled to a pipe **126** that routes the condensate to the condensate trap as shown in FIG. **1A****.** Other routing structures may be used. For example, often the condensate trap is located below the condensate producing component and collects the condensate directly.

Once collected, the condensate is routed to the aerosol generator **118** via the conduit **120.** In some instances, a condensate pump **122** is used to move the condensate between the condensate trap and the aerosol generator. The condensate pump pressurizes the condensate fluid, directing it through the conduit, vertically if necessary. This pressurization may also allow the condensate to be dispersed within the aerosol generator via a sprayer (not shown) or other fitting, which may aid in the generation of aerosol from the condensate.

The drive circuit **124** is used to energize the aerosol generator, which creates an aerosol with negative ions from the condensate. The aerosol is mixed with the conditioned air and directed to a conditioned space **114.** Often the aerosol is mixed with the conditioned air after the conditioned air has been through various HVAC equipment **106,** allowing the aerosol to bypass some or all of the HVAC components and be routed directly to the conditioned space.

The HVAC equipment **106** may include an indoor unit, an outdoor unit, and a refrigerant loop extending between the indoor unit and the outdoor unit. The indoor unit may include a furnace or air handler, an indoor refrigerant heat exchanger or evaporator to condition air (heat or cool), and the fan **108** to circulate or otherwise provide the conditioned air to the conditioned space. The outdoor unit may include an outdoor fan and an outdoor refrigerant heat exchanger or condenser, and the refrigerant loop may extend between the indoor and outdoor refrigerant heat exchangers. In more complex systems, often used in higher load applications such as commercial buildings, additional equipment such as chillers, cooling towers, water source heat pumps, etc. may also be included within the HVAC system. In addition, in some examples, the HVAC equipment includes a refrigeration unit that provides lower temperature conditioning, potentially for food storage and/or freezing. The refrigeration units may also be transport refrigeration units, where the refrigeration unit is located on a portable trailer, and the refrigeration unit provides cooling to the conditioned space within the trailer.

The HVAC equipment **106** may further include a cooling element **110,** such as cooling coils used to cool and condition the air provided to the various zones. In some examples, the system utilizes refrigerant cooling coils, and provides a direct exchange of heat between the conditioned air and the refrigerant. Other cooling elements or fluids may also be utilized, including hydronic/water coils, etc. Similarly, the HVAC system **104** may utilize a variety of different heating elements **112,** such as electric, gas, refrigerant heated coils, hydronic/water heated coils, etc. In some examples, the cooling element **110** and the heating element **112** may be the same coil within an air handling unit or a heat pump system. The HVAC equipment may be arranged to provide staged capacity relief. In some examples, multiple cooling and/or heating elements are provided to allow the HVAC system to provide staged capacity to the system in either cooling or heating mode. In some examples, the fan is similarly configured to operate at set stages to provide staged capacity to the zones calling for conditioning relief. In other examples, the cooling element, heating element, and/or fan may be continuously adjusted over a set range or range(s) allowing the system to adjust the capacity provided in a continuous or substantially continuous manner over that range.

In some examples, the HVAC system **104** may include an air circulation path with supply air ducts including a main supply air duct **128** and branch supply air ducts **130** through which the conditioned air from the HVAC equipment **106** is provided to the conditioned spaces **114A, 114B, 114C.** As shown in FIG. **1A****,** in some examples, the air purifying device **102** is coupled to the main supply air duct, allowing the aerosol generated from the condensate to combine with conditioned air within the supply air duct. In addition, in some instances, the HVAC system includes a branch supply duct **132** directing air to an outside environment **134.** To control airflow to the zones, the HVAC equipment may include zone dampers **136.** The air circulation path also includes return air ducts including space return air ducts **138** and a main return air duct **140** operably coupleable with the HVAC equipment, and through which air in the zones of the conditioned space is returned to the HVAC equipment as return air. In some examples, the HVAC system includes a supply of outdoor air **142** directed through a return air duct **144,** and in some examples, this outdoor air is coupled to the main return air duct **140.**

The HVAC system **104** in FIG. **1A** includes control circuitry **146** operably coupled with the HVAC equipment **106** and the air purifying device **102.** The control circuitry is configured to control the air purifying device, adjusting both the volume and type of aerosol produced. The control circuitry is also configured to control the HVAC equipment to provide the conditioned air to heat or cool the conditioned space **114,** or in some examples, spaces **114A, 114B, 114C** of the conditioned space. In some examples, the control circuitry is configured to control the air purifying device and/or the HVAC equipment based on environmental feedback from one or more sensors operably coupled to the control circuitry. In this regard, the HVAC system may include at least one of a temperature sensor **148,** a humidity sensor **150,** and an occupancy sensor **152,** or potentially other sensors such as an air quality sensor.

In some examples, the HVAC system **104** may include a conditioned air temperature sensor **148** in the conditioned space **114** configured to measure temperature in the conditioned space. In some examples, as shown, one or more of the spaces **114A, 114B, 114C** may include a respective conditioned air temperature sensor for the space and configured to measure temperature in the space. In some examples, the control circuitry **146** (or some portion of the control circuitry) and the conditioned air temperature sensor may be co-located in a thermostat **154.** In other examples, the control circuitry and the conditioned air temperature sensor may be separated and connected by wired or wireless communication; and in some of these examples, the conditioned air temperature sensor may be co-located with other electronic devices or circuitry such as a display device, network adapter, or the like.

Similarly, the humidity sensor **150** and the occupancy sensor **152** may be co-located within a central device such a thermostat **154.** In other examples, some or all of these sensors may be located as separate components or part of another device. The occupancy sensors may provide an indication of the number of people within a space. These occupancy sensors may be any type of sensor that provides this functionality including, carbon dioxide (CO2) sensors, motion sensors, video fields, thermal imaging cameras, temperatures sensors, or others, some of which may be combined with analytics to provide more accurate occupancy information. Humidity sensors **150** may also be used to provide humidity information, e.g., relative humidity, web bulb temperature, etc. The control circuitry may further be coupled to auxiliary devices, such as operating schedules, occupancy preference, real-time clocks, energy rate schedules, etc.

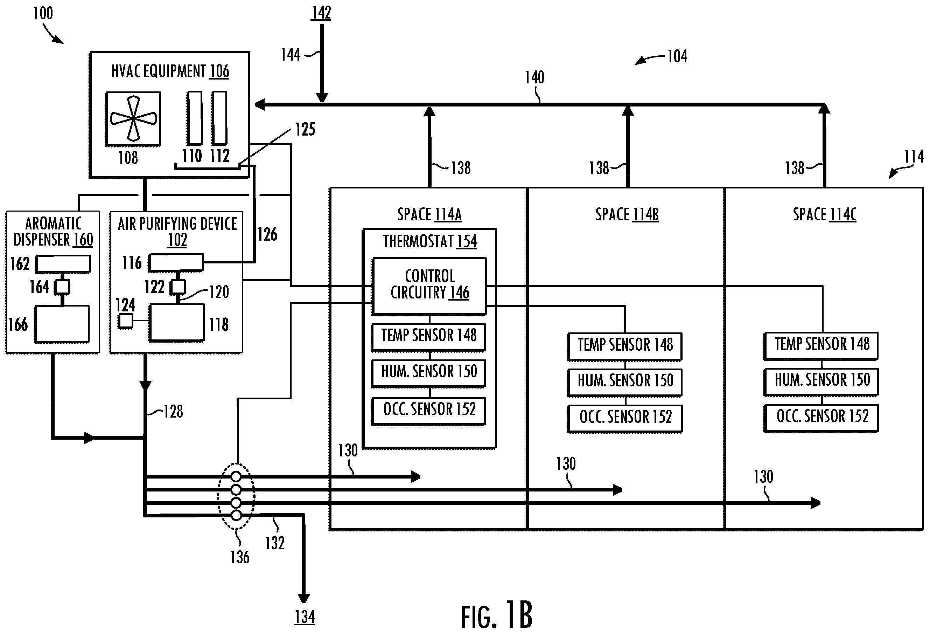

FIG. **1B** shows an example embodiment where the system discussed above in regard to FIG. **1A** that also includes an aromatic dispenser **160** that dispenses an aromatic aerosol which may be combined with the conditioned air and delivered to the conditioned space(s). The aromatic dispenser may include a dispenser **162** that dispenses an aromatic **164** that is aerosolized by an aromatic aerosol generator **166.**

The aerosol dispenser **160** may include a dispenser **162** that dispenses an aromatic **164.** The aromatic may be combined with a fluid to create an aromatic fluid. The aromatic fluid is then supplied to an aromatic aerosol generator **166** that produces an aromatic aerosol that may be combined with the conditioned air provided by the HVAC system for delivery to the conditioned space.

In some examples, the HVAC system **104** includes control circuitry **146** operably coupled with the HVAC equipment **106,** the air purifying device **102,** and the aromatic dispenser **160.** The control circuitry is also configured to control the aromatic dispenser to activate or deactivate it, adjust the rate aromatic aerosol is produced, adjust the type of aromatic dispensed, or other functionality including the functionality discussed above with regard to the air purifying device **102.**

In some examples, this aerosol dispenser **160** is coupled to the air purifying device **102** to dispense an aromatic that is combined with the condensate and aerosolized by the aerosol generator. In some examples, the aerosol dispenser may be a separate device and include its own aerosol generator for generating an aromatic aerosol.

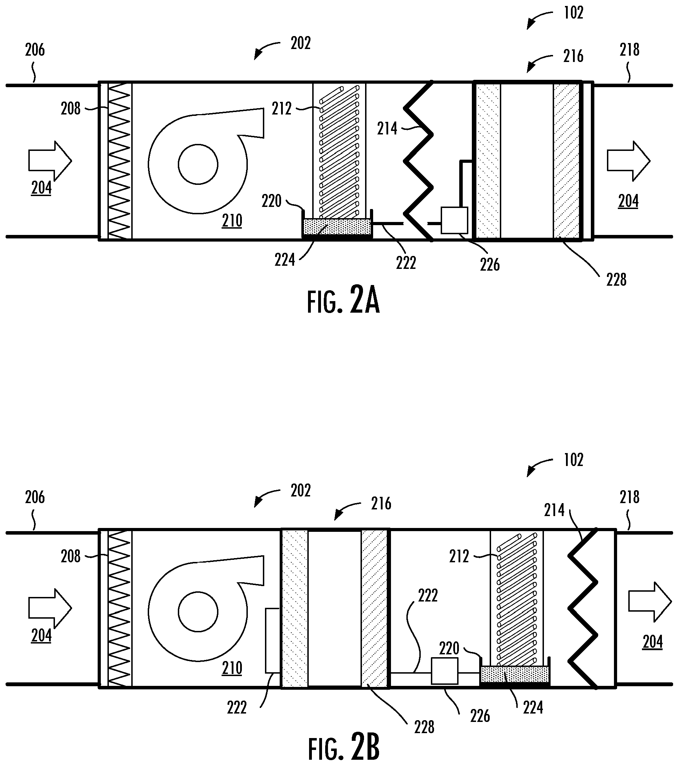

FIG. **2A** shows a schematic of the components that may comprise the air purifying device **102** along with components of the HVAC system **104.** In this example, these components are located within an air handling unit **202** and the conditioned air path is indicated by arrow **204.** Return air from a conditioned space and/or outdoor air are directed to the air handling unit via return air duct **206.** In this example, the air first passes through a mechanical filter **208,** before entering an inlet of the fan **210.** The fan pressurizes the air and directs it to the cooling element **212,** and in this example, through a heating element **214** as well. The conditioned air is then directed through the aerosol generator **216,** and finally into the supply air duct **218,** which directs the air into a conditioned space (not shown).

In other implementations, the air handling unit **202** may be located vertically with cooling element **212** and/or the heating element **214** disposed above the fan **210.** In other examples, the fan is located down stream of either the cooling element or the heating element. In still other examples, the cooling element and/or the heating element may be disposed within a fluid duct of the air handling unit and may also be selectively removable from the air handling unit. It is appreciated that more or less of these components may be utilized, and that they may be arranged in different configurations provided they provide the functionality described herein.

Condensate trap **220** collects condensate **224** and is shown coupled to the cooling element **212** in the depicted example in FIG. **2A****.** A conduit **222** couples the condensate trap to the aerosol generator **216,** allowing the condensate created by the HVAC system **104** to be supplied to the aerosol generator. It is appreciated that the condensate trap may be associated with any condensate producing components within the overall device, including but not limited to, the heating element **214,** the cooling element **212,** or other components. In the depicted example, a condensate pump **226** is also used to move the condensate between the condensate trap and the aerosol generator.

While the condensate trap **220** is depicted as a U-shaped trap in this example, the condensate trap may comprise any suitable type of trap in other examples (e.g., P-shaped or S-shaped). The condensate trap may be configured to receive condensate from a drain pain **125** of an air handler, as illustrated schematically in FIG. **1A****.** For example, the drain pan may generally be arranged such that condensation may flow away from the drain pan and into the pipe **126** through the force of gravity, where it is directed into a condensate trap. In some examples, the condensate trap is the drain pan.

As shown in the depicted embodiment in FIG. **2A****,** the various components are located within an air handling unit **202.** This configuration has various advantages, particularly due to its compact size. In addition, the components of the air purifying device **102** may be incorporated within an existing supply air path, potentially a supply air duct **218.** In some examples, the air purifying device is associated with a ductless HVAC system **104** and located either within the ductless unit itself, coupled to the unit, or coupled to the conditioned space. In these examples, some or all of the various components within the air purifying device may be located within a plenum or other air channel directing the supply air out of the device. In other examples, one or more of these components may be located outside the supply air duct, or even outside the HVAC system.

FIG. **2B** shows an alternative configuration with the same components. In this example, the aerosol generator **216** is located upstream of the cooling element **212** and the heating element **214.** This configuration allows the aerosol generator to provide aerosol over these components, and in some examples, the aerosol generator cleans and/or disinfects the cooling element and/or the heating element.

In some examples, the HVAC equipment **106** includes conditioning coils and the aerosol includes droplets of the condensate. In these examples, the fan **210** is operable to direct the aerosol over the conditioning coils on which those of the droplets that contact the coils freeze and thereby form a layer of frost on the coils. This may be based on the configuration of the devices, e.g., FIG. **2B** locates the aerosol generator **216** upstream and proximate the cooling element **212.** This configuration allows the aerosol generator to direct the aerosol onto the coils of the cooling element. The air handling unit **202** may also be operating in cooling mode, which in some examples, may result in the cooling elements having a surface coil temperature low enough to form frost on the coils. The frost may be formed from the aerosol, and in some examples, the aerosol forms a layer of frost on the coils of the cooling element. The layer of frost that includes frost from the aerosol may disinfect the coils, and in some examples, this frost also disinfects the air that passes through the coils. Other configurations may also be arranged and utilized.

In some examples, the components described herein may be included as a kit and may be added to an existing HVAC system **104.** This kit may include some or all of the following components: the condensate trap **220,** the conduit **222,** the condensate pump **226,** and the aerosol generator **216,** all of which may be the same or similar to the components described herein. In some examples, these components also include brackets or extendable features allowing them to couple to and/or fit within the existing HVAC system.

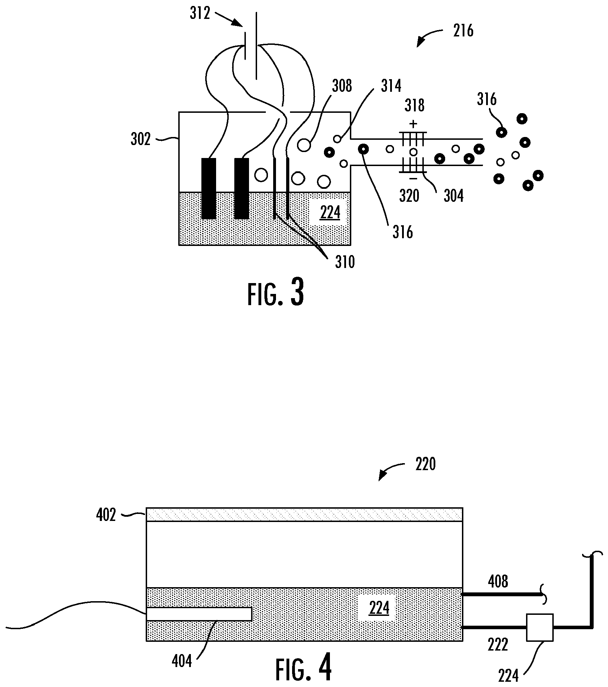

FIG. **3** shows an example illustration of an aerosol generator **216** that may be used. The aerosol generator **216** may use various techniques to create an aerosol including negative ions from the condensate. In some examples, the aerosol generator uses water shearing, potentially the Lenard Effect, to generate the aerosol and the negative ions. In some examples, a field charger may create an electrical charge for ionizing the aerosol. This process at times is referred to as a corona effect. Other processes may be used for generating the aerosol and the negative ions, and these various processes may be used together in some instances. In some examples, this process also ionizes the fluid used to create the aerosol. In these examples, the aerosol generator uses the processes to generate the aerosol, and the fluid that undergoes these processes but does not aerosolized becomes an ionized liquid.

The aerosol generator **216** shown in FIG. **3** uses both water shearing and a field charger. In the depicted example, the aerosol generator includes a bulk charging unit **302** and an electric field module **304.** The condensate **224** may collect within the bulk charging unit to be generated into an aerosol **308.** The bulk charging unit in this example further includes two piezo transducers **310** coupled to a drive circuit **312,** which may be used to generate an aerosol by vibrating the condensate. In this example, the piezo transducers are positioned to engage with the condensate, and the drive circuit drives the piezo transducers, causing them to vibrate. This vibration shears and breaks up the condensate, which generates aerosol, typically in the form of water droplets **314,** potentially liquid nanoparticles, and negative ions **316,** which may be in the form of ionized water droplets or vapor. In some examples, these droplets form negative ions either directly from the energy provide via the transducers or through collisions with each other. In some examples, sprayers, or tortuous passages that shear the fluid, create an aerosol and negative ions.

In some examples, the aerosol generator **216** includes a mesh (not shown) coupled to piezo material and/or transducers. In these examples, the vibration of the piezo material causes the mesh to vibrate, which breaks up the condensate, creating the aerosol and negative ions. Other features or configurations may be used to vibrate and shear the condensate to create the aerosol and negative ions.

The drive circuit **312** is configured to drive the piezo transducer(s) **310,** or in some examples a mesh, in a variety of different ways. The drive circuit may drive the piezo transducers or materials to generate ultrasonic waves within the condensate. These ultrasonic waves may vary in frequency and intensity, which may impact the aerosol generated. In some examples, the drive circuit drives the pizeo at a power level and frequency to generate very small particles such as liquid microparticles or even the smaller liquid nanoparticles. In these examples, the drive circuit may generate 70% or more liquid nanoparticles. In some of these examples, the amount and/or percentage of nanoparticles generated varies. In some examples, the drive circuit generates microparticles, which may be generated with the nanoparticles.

In some examples, were the system includes multiple piezo transducers or materials, the drive circuit **312** energizes the piezo transducers **310** or material to synchronously vibrate. This may include energizing these piezo transducers and/or material at the same frequency and power level. In other examples, the drive circuit energizes the piezo transducers or material to asynchronously vibrate. In these examples, this may include energizing these piezo transducers and/or materials at the different frequencies and/or power level. This asynchronous vibration may direct vibration energy through the condensate in a given direction or pattern, facilitating different amounts of aerosol generation, different sized droplets, and/or additional ionized aerosol particles.

In some examples, the aerosol generator **216** further includes an electric field module **304** arranged downstream from the bulk charging electrolytic unit **302.** The electric field module may provide an electrical charge to the aerosol generated by the bulk charging electrolytic unit. In some examples, the electric field module may include a positive electrode **318** and a negative electrode **320.** When the electric field module is energized, these electrodes energize the aerosol **308** and surrounding space. In some examples, this electric field charges the water aerosol droplets **314,** creating more negative ions **316.** In some examples, the aerosol generator is configured as an enclosed system, in which case aerosol generated by the system is comprised of only (or substantially only) aerosol generated from the condensate, which allows the field charger to energize primarily (or exclusively) the aerosol generated from the condensate. In some examples, the electric field module is a field charger and uses the corona effect to generate the electric field and in turn the negative ions.

In some examples, the aerosol generator **216** also generates an ionized liquid using the above process. This ionized liquid may be produced as a result of the condensate or other fluid that undergoes the shearing and/or field charging process but is not aerosolized.

In some examples, the aerosol generator **216** further includes a filter **260** as shown in FIG. **2A****.** This filter may be positioned downstream from the bulk charging electrolytic unit **302** and an electric field module **304.** In some examples, the filter engages with the aerosol and negative ions after they have combined with the conditioned air. This may allow the aerosol and negative ions to interact with any pollutants that may be present in the conditioned air prior to passing through the filter. In some instances, the filter includes a charge, potentially a positive charge, which attracts any pollutants that have been negatively charged by the ions generated within the aerosol generator. In these instances, the filter may remove these pollutants from the air stream. In some examples, the filter is sized to remove any larger aerosol particles which may be undesirable in the conditioned air supplied to the spaces.

FIG. **4** shows an example of a condensate trap **220** that may be used in some implementations of the present air purifying device **202.** In the example depicted in FIG. **2A****,** the condensate trap is coupled to the cooling element **212,** however, as discussed above, the condensate trap may be coupled to any condensate generating component. In addition, the condensate trap may include additional features to filter and disinfect the condensate **306,** including a particle filter **402** and an ultraviolet light source **404.**

The particle filter **402** may be one or more filters designed to remove solid particles from the condensate **306.** These filters may be removable filters, and potentially disposable. In some examples multiples filters are arranged to clean and purify the water collected within the condensate trap.

The ultraviolet light source **404** may comprise a plurality of lights energized to disinfect the condensate. In the example depicted in FIG. **4****,** a single light is submerged within the condensate **306.** However, multiple lights may be used and arranged in a different matter, such as over the condensate, coupled to the particle filter, etc. In addition, the particle filter **402** and/or ultraviolet light source may be coupled to the condensate trap **220** discharge and/or conduit **222.** In some examples, the condensate trap includes an anti-microbial material that prevents mold or other contaminants to grow on the condensate trap. Some examples only include one of these components, and other examples include none at all.

In some examples, the condensate trap **220** includes an outlet coupled to the conduit **222** for routing the condensate **306** to the aerosol generator **216.** The conduit **222** may be coupled to a condensate pump **406** that pressurizes the condensate, moving it from the condensate trap to the aerosol generator. The condensate trap may further include at least one additional outlet connected to a discharge drain **408.** The discharge drain may be utilized when the aerosol generator is not in use and/or when the condensate collected by the condensate trap exceeds the demands of the aerosol generator. In some examples, the discharge drain may be coupled to the condensate trap above the outlet for the conduit, which may allow the discharge drain to serve as the overflow drain for the condensate trap.

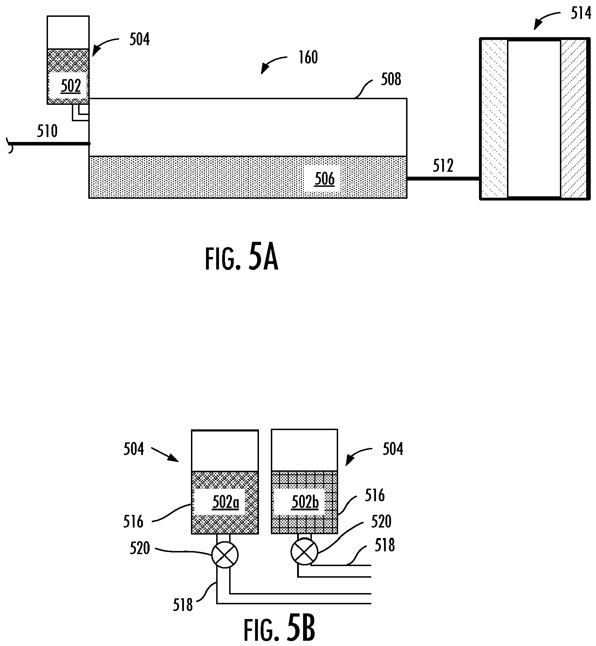

FIGS. **5A** and **5B** show an example illustration of an aromatic dispenser **160** that may be used in some implementations of the present air purifying device **202.** The aromatic dispenser may be configured to supply an aromatic **502** that can be combined with the conditioned air provided by the HVAC system **104.** The combination of the aromatic and the conditioned air may be delivered to a conditioned space, and in some examples, the aromatic and the conditioned air are combined before they reach the conditioned space. The aromatic dispenser include various features discussed above or may be a separate device. In some examples, the aromatic dispenser is incorporated within various features discussed above, for example, the aromatic dispenser may be included part of the aerosol generator **216.**

FIG. **5A** shows an example illustration of the aromatic dispenser **160,** which may include some or all of the following components: an aromatic **502,** a dispenser **504,** a fluid **506,** a fluid storage tank **508,** a fluid supply **510,** an aromatic conduit **512,** and an aromatic aerosol generator **514** for generating an aromatic aerosol. Each of these components and how they interrelate are discussed more below.

The example shown in FIG. **5A** is illustrative of how various components of the aromatic dispenser **160** may interact. In this example, the aromatic **502** is supplied into the fluid **506** using a dispenser **504.** This fluid is contained within the fluid storage tank **508,** and in this example, the aromatic and the fluid combine within the fluid storage tank to create an aromatic fluid. The aromatic fluid may then be routed via the aromatic conduit **512** to an aromatic aerosol generator **514** configured to energize the aromatic to generate an aromatic aerosol. The aromatic aerosol may be combined with the conditioned air and supplied to a conditioned space.

FIG. **5B** show example illustrations of the dispenser **504,** and in this example, the aromatic dispenser **160** includes multiple dispensers. In the example shown each dispenser includes a different constituent aromatic, e.g., one dispenser includes aromatic **502a** and the other includes aromatic **502b**. Other examples may include multiple dispensers where one or more dispensers include the same constituent aromatic. In addition, while two dispensers are shown, it is understood that more or less dispensers may be used. It is also understood that this illustration is only an example configuration of dispensers that may be used to dispense and/or combine aromatics.

In the example shown in FIG. **5B****,** the dispenser includes a container **516** for housing the aromatic **502** and an injector **518** for supplying the aromatic into the fluid **506** (shown in FIG. **5A**). The dispenser **504** may direct the aromatic **502** from the container via the injector into the fluid **506.** In this example, the fluid is contained within the fluid storage tank **508** where the fluid and the aromatic may be combined. In some examples, the dispenser may supply the aromatic into the fluid supply **510** (e.g., a water line), which may feed into the fluid storage tank. In other examples, the dispenser may supply the aromatic into the discharge from the fluid storage tank. In some examples, a fluid storage tank may not be necessary.

In some examples, the aromatic dispenser **160** uses the dispenser **504** to supply the aromatic **502** into the condensate **224** to produce an aromatic condensate. In these examples, the fluid storage tank **508** may be the condensate trap **220,** and the aromatic is combined with the condensate in the condensate trap. In some examples, the aromatic may be dispensed into a water supply line that feeds into the condensate trap, which allows the aromatic, the water, and the condensate all to be combined together. In some examples, the aromatic is dispensed into the condensate after the condensate has left the condensate trap.

In some examples, the aromatic dispenser **160** may combine multiple different aromatics **502.** For example, the aromatic dispenser may supply two or more constituent aromatics **(502a** and **502b)** that are individually combinable with the conditioned air. In these examples, the aromatic dispenser may include one or more dispensers **504** with a given aromatic (e.g., lemon scented aromatic) and one or more other dispensers with another, different aromatic (e.g., lilac scented aromatic). The system may further control the various dispensers to only dispense a given constitute aromatic at a time, e.g., only lemon scent at a first time and only lilac scent at a second time.

In other examples, the aromatic dispenser **160** supply two or more constituent aromatics (**502a** and **502b**) that are collectively combinable with the conditioned air. In these examples, the system may dispense two or more constitutes aromatics at the same time, e.g., combining lemon scented aromatic with the lilac scented aromatic, to create an aromatic that is different form either constitute aromatic on its own (e.g., a lemon-lilac scented aromatic). This may allow the aromatic dispenser to create multiple aromatics from a limited number of dispensers.

The control circuity **146** may control these dispensers **504** to allow the aromatic dispenser to supply different aromas for different purposes. For example, aromatic dispenser may supply aromatics based on a schedule. For example, one of the aromatics may be supplied during the daytime and another may be supplied at night. The aromatics (or lack thereof) may also correspond to various events such as a meal, a meeting, a cleaning event, an alarm event, a bedtime, etc. In some examples, the aromatic may be based on a user selection, potentially provided through the controller, and the user may make changes to the aromatic at any time. Various methods may be used for controlling these dispensers may also be used.

The control circuity **146** may also control the rate at which the aromatic is supplied. For example, the dispenser **504** may control the rate at which aromatic is supplied into the fluid. In some examples, this rate may be adjusted based on inputs to the system including various sensors, users input, or other system operations.

In some examples, the dispenser **504** includes a modulating valve **520** or variable opening for adjusting the supply, and/or rate of supply, of the aromatic **502** into the fluid **506.** Any standard modulating valve (e.g., ball valve, butterfly valve, etc.) or standard technique for adjusting flow may be used. In addition, in some examples, the dispenser (and potentially the modulating valve or adjustable opening) is connected to the control circuity **146** and may be controlled based on various inputs such as system operating parameters, sensors, user input, etc. to adjust the flow or supply of aromatic form the dispenser(s).

The aromatic **502** used with the aromatic dispenser **160** may come in a variety of different forms. For example, a water-soluble liquid may be used and included within the container **516** of the dispenser **504.** Other examples may use aromatics that are water soluble tablets, which may also be located within the container of the dispenser **504.** In examples that use water soluble tablets, the injector **518** may be a lever or a sliding wall that moves the water soluble tablets from the container into the fluid. Other mechanisms may also be used.

In some examples, the aromatic **502** is directly dispensed by an individual, potentially a technician, into the fluid to be supplied to the aromatic aerosol generator **514.** In these examples, the tablet aromatic, for example, may be dispensed into a water source or the condensate trap directly by an individual.

In some examples, the aromatic dispenser **160** may also include a fluid storage tank **508.** This fluid storage tank may retain the fluid **506** to be combined with the aromatic **502.** In some examples, the fluid storage tank may also serve to combine and/or mix the fluid with one or more constitute aromatics. In some examples, a fluid supply **510** is coupled to fluid storage tank to provide a consistent source of fluid. In some examples, the fluid is water and the fluid supply is a water line. In some examples, the fluid storage tank is the condensate trap, and in some of these examples, the fluid storage tank may not include a fluid supply.

In some examples, the aromatic dispenser **160** may also include an aromatic conduit **512** to supply the fluid combined with the aerosol to the aromatic aerosol generator **514.** In some examples, the aromatic conduit is coupled to the fluid storage tank **508** and directs the fluid to the aromatic aerosol generator. In some examples, the aromatic conduit directs the aromatic directly from the dispenser **504** to the aromatic aerosol generator. In some examples, a pump similar to the pump **226** is necessary to move the fluid combined with the aerosol through the aromatic conduit. In some examples, the aromatic conduit is conduit **222,** and in some examples the aromatic conduit is substantially the same as conduit **222.**

In some examples, the aromatic aerosol generator **514** is the same or substantially similar to the aerosol generator **216** discussed above. The aromatic aerosol generator may include some or all of the components of aerosol generator **216.** In some examples, the aromatic dispenser is coupled to the condensate trap **220.** In these examples, the dispenser **504** may supply the aromatic to be combined with the condensate in the condensate trap. The condensate trap may serve as the fluid storage tank 508, and in these examples, the aerosol generator coupled to the condensate trap may serve as the aromatic aerosol generator for the aromatic dispenser. In other examples, the aromatic dispenser may be a separate system and may include a separate aromatic aerosol generator **514** for generating the aromatic aerosol.

In some examples, the aromatic aerosol generator **514** is controlled such that it only generates aromatic aerosol when the aerosol includes negative ions. In examples that include both an aerosol generator **216** and a separate aromatic aerosol generator **160,** this control may include controlling the aromatic aerosol generator such that it only operates when the aerosol generator is operating. In embodiments, where the aerosol generator and the aromatic aerosol generator are the same device, this control may include controlling the dispenser **504** to only dispense aromatic **502** when the transducers **310** are vibrating at a frequency sufficient to produce negative ions.

Returning to FIGS. **1A** and **1B****,** the HVAC system **104** may include an air circulation path comprising the supply air ducts **128, 130, 132** and the return air ducts **138, 140, 144,** which directs the conditioned air. In some examples, the HVAC system may include only supply air ducts or only return air ducts, or no ducts at all. Regardless, the conditioned air in this path may be combined with the aerosol generated by the air purifying device **102,** and in some examples, the aerosol includes negative ions. The air circulation path may direct this conditioned air, along with the aerosol, into one or more conditioned spaces **114.** The air circulation path may further direct the conditioned air with the aerosol to targeted locations that may be particularly susceptible to air or surface contaminants. These spaces may include restrooms, dining rooms, meeting rooms, or in some cases specific locations within a room such as a water closet, work out space, or other locations. In some examples, as shown in Fig. **1****,** the air circulation path may direct conditioned air with the aerosol to an outdoor environment **134,** potentially the ingress or egress locations for a building or conditioned space. This air may also be directed to outdoor congregation locations, or even the outdoor environment generally.

In some examples, the aromatic dispenser **160** is coupled to the HVAC system **104** and supplies the aromatic aerosol into the air circulation path to be combined with the conditioned air. In some examples, the aromatic aerosol is supplied into a supply air duct. In some examples, the aromatic aerosol is supplied via the air purifying device.

In some examples, control circuitry **146** is coupled to the HVAC system **104,** the air purifying device **102,** and/or the aromatic dispenser **160.** The control circuitry may be configured to control and monitor any of components discussed above. In some examples, the control circuitry is configured to control these components in the following manner.

As shown in FIGS. **1A** and **1B****,** the control circuitry **146** may be operatively coupled to the HVAC system **104,** the air purifying device **102,** and/or the aromatic dispenser **160.** In some examples, the control circuitry controls the air purifying device based on the occupancy of a given space or zone. In these examples, the control circuitry receives an indication of the occupancy of a given space or zone from one or more occupancy sensors **152.** The control circuitry then controls the generation of the aerosol based on the occupancy measurements received. In these examples, the control circuitry may seek to initiate or increase the quantity of aerosol to a space based on the occupancy of the space. For example, the control circuitry may increase the quantity of aerosol generated as the occupancy increases, or conversely decrease the quantity of aerosol as occupancy declines. In some examples, a minimum and/or maximum aerosol quantity is set for the system.

In some instances, the control circuitry **146** may control the air purifying device **102,** and potentially HVAC equipment **106,** based on the occupancy measurements for a given space independent of any cooling or heating load. For example, in some instances, the occupancy sensor **152** may indicate a space is occupied, however, the temperature sensor **148** or other conditioning sensors indicates that the space is within acceptable temperature parameters. In these instances, the control circuitry may initiate an air cleaning mode, whereby the air purifying device generates aerosol and negative ions, and the fan **108** circulates air combined with the aerosol and negative ions to the space without conditioning the air, e.g., without activating the heating or the cooling element. In some of these examples, a separate water supply line (not shown) may be coupled to the condensate trap in the event insufficient condensate is available.

In some examples, the control circuitry **146** receives scheduling information for one or more conditioned space(s) or zone(s). This information may be in any form including an estimate of occupancy, heating/cooling load, or otherwise, for a given space/zone at a given time. In some examples, this scheduling information is arranged in time intervals. In these examples, the control circuitry may control the aerosol generator based on, at least in part, the expected load for at a given time interval.

For example, the expected conditioning load for one-time interval, potentially a first time interval, may be less than expected conditioning load for another time interval, potentially a second time interval. This change in load may indicate that the space will be occupied during the second time interval. In these instances, the control circuitry may increase the quantity of aerosol generated during the first time interval in advance of the second time interval. In some instances, this may include initiating an air cleaning mode if the space or zone does not have any heating or cooling load at that time. This may allow for the system to clean and disinfect the conditioned space in advance of a given event, e.g., a meeting, etc.

In some instances, the production of aerosol is continued for some or all of the event. In some examples, the aerosol generation and distribution is initiated near or at the end of the event or time interval. In some examples, the aerosol is generated and directed into the space for a short period of time, such as 5 minutes, 10 minutes, or 15 minutes. In other examples, the time period is longer, one hour or more. In some examples, the aerosol is generated and directed into the space until the air within the room is partially or fully replaced.

The control circuitry may also control the aromatic dispenser in a similar manner in response to occupancy measurements, and in some examples, the air purifying device and the aromatic dispenser are controlled jointly in response to occupancy measurement.

In some examples, the control circuitry **146** includes humidity sensors **150** and controls the air purifying device **102** and/or HVAC system **104** based on humidity information received from the humidity sensors. The humidity sensors in these examples may be positioned within a space to measure the humidity level generated by conditions of the space, including any outdoor air provided to the system along with the humidity generated by the air purifying device. The control circuitry may control the HVAC system based on these humidity measurements.

For example, the control circuitry **146** may ensure the humidity levels within a space are within a given humidity range. If the humidity measured in the space is over an acceptable or desired humidity level, the control circuitry may seek to dehumidify the conditioned air supplied to the space. In some of these examples, this may involve increasing the cooling relief applied to the conditioned air by the cooling element **110.** If this increase in cooling relief impacts the temperature of the space in an undesirable manner, the heating element **112** may be used, potentially in a reheat mode. In some examples, the control circuitry decreases or shuts off the amount of aerosol generated **118** by the air purifying device in response to high humidity measurements. In instances where the humidity level is measured to be below a desired or acceptable humidity range, the control circuitry may initiate or increase the volume of aerosol generated by the air purifying device.

In some examples, the control circuitry **146** may adjust a rate at which aerosol is generated by the air purifying device **102** based on the measurements of the humidity level. In these examples, the control circuitry may decrease the rate at which the aerosol is generated when the system receives an indication that a high humidity level is detected. For example, a humidity sensor in a conditioned room may detect a higher level of humidity than desired, or the humidity level may be above a given threshold or range. In response to this humidity measurement, the control circuitry may control the air purifying device to decrease the aerosol being produced. The control circuity may also operate the system conversely to increase the supply of aerosol provided by the air purifying device in response to a low level of humidity being detected, e.g., a level of humidity below a threshold level, range or a desired level.

In some examples, the control circuitry **146** is configured to adjust a particle size of the aerosol generated by the air purifying device **142** based on the measurements of the humidity level. In these examples, the control circuitry may decrease the particle size of the aerosol generated by the air purifying device in response to the system receiving an indication that a high humidity level is detected. For example, a humidity sensor in a conditioned room may detect a higher level of humidity than desired, or the humidity level may be above a given threshold or range. In response to this humidity measurement, the control circuitry may control the air purifying device to decrease particle size of the aerosol being produced. For example, the air purifying device may be controlled to adjust the frequency or power provided to the transducers to reduce the size of the particles produced and/or increase the percentage of smaller particles (e.g., microparticles or nanoparticles) produced. The control circuity may also operate the system conversely to increase the particle size of the aerosol provided by the air purifying device in response to a low level of humidity being detected, e.g., a level of humidity below a threshold level, range or a desired level.

The control circuitry may also control the aromatic dispenser in a similar manner in response to humidity measurements, and in some examples, the air purifying device and the aromatic dispenser are controlled jointly in response to humidity measurement.

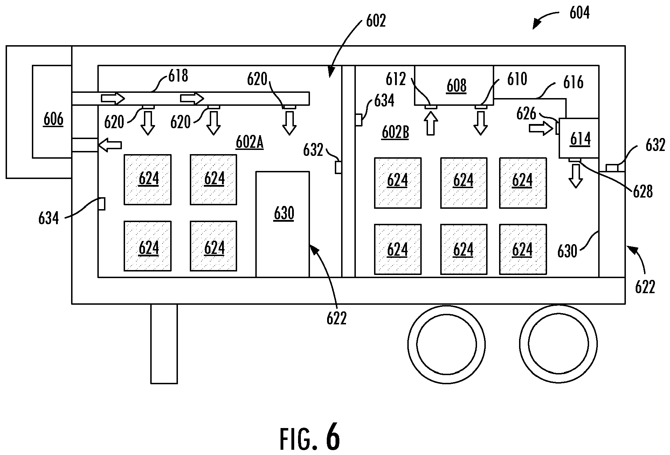

FIG. **6** shows an example illustration of the air purifying system **100** used to condition and disinfect a conditioned space. The depicted example includes a conditioned space **602** with two different spaces **602A & 602B,** which may also be referred to as compartments. Both spaces are included within a portable trailer **604** in the depicted embodiment. One of the spaces **602A** includes a wall mounted air handling unit **606.** This air handling unit includes a ducted supply path that directs the conditioned air from the air handling unit **606** into a certain location within the space **602A.** The other conditioned space **602B** includes a roof mounted air handling unit **608** that circulates conditioned air between a supply air register **610** and a return air register **612** to condition the space **602B.** The roof mounted air handling unit **608** is coupled to air curtain **614** via a conduit **616.** In some examples, one or both of air handling units **(606** and **608)** are refrigeration units that produce low temperature conditioning to spaces **602A & 602B,** respectively. In some examples, these refrigeration units provide temperatures sufficient to freeze the contents within the spaces.

Each of the air handling units **(606** and **608)** may include some or all of the components discussed above in connection with air handling unit **202.** For example, the wall mounted air handling unit **606** may include an aerosol generator **216,** a fan **210,** and other components, e.g., cooling element **212,** heating element **214,** etc. Similarly, the roof mounted air handling unit **608** may include a fan **210,** a cooling element **212,** a heating element **214,** etc. In some examples, the roof mounted air handling unit may include an aerosol generator **216** and in other examples it may not.

In some examples, these air handling units **(606** and **608)** are refrigeration units, where low temperature conditioning is provided, potentially freezing conditions. In these examples, the air handling unit(s) may include all of the same features discussed above, and may be designed for the lower temperature application. These units may also include additional equipment coupled to (or include) in the unit, and the additional equipment may include a compressor and an additional heating exchanger(s) to provide a complete refrigeration circuit. These refrigeration units may also be configured to provide the low temperature cooling, for example, by including a certain type of refrigerant, valve setting, control features, etc., directed to lower conditioning temperatures. In addition, in the depicted embodiment the refrigeration units would be considered transport refrigeration units because they are located on a portable trailer. In these examples, the full refrigerant circuit and related components, e.g., condenser, evaporate, metering device, compressor, etc., may be portable with the trailer.

The air curtain **614** in the depicted embodiment includes a fan **210** (not shown in FIG. **6****)** and an aerosol generator **216** (not shown in FIG. **6****).** In the depicted example, the conduit **616** is coupled to a component of the roof mounted air handling unit **608** that collects condensate **224,** potentially a condensate trap similar or the same as condensate trap **220.** The conduit routes the condensate from the condensate trap to the aerosol generator within the air curtain.

In some examples, the HVAC system **104** is set up in an installation with a duct that defines a pathway to a location proximate an ingress to the conditioned space. In these examples, a fan **210** may operate to move the aerosol through the duct, toward the location and thereby the ingress to the conditioned space.

The HVAC system **104** shown in FIG. **6** providing conditioning to space **602A** provides an example depiction. In this example, the wall mounted air conditioning unit **606** may include a fan **210** (not shown in FIG. **6**) and an aerosol generator **216** (not shown in FIG. **6**), and it is connected to a supply air duct **618** with air registers **620.** The supply air duct routes the conditioned air and aerosol generated at the aerosol generator to a location within the space, likely a location where the conditioning and/or disinfecting will be most impactful. In the depicted embodiment, the duct routes the combined condition air and the aerosol to a location proximate the ingress **622** to space **602A.** This may allow the system to condition and disinfect the air entering the space. The ductwork may also include ducts that route the conditioned air and aerosol to other locations within the space, as well as multiple locations within space. For example, ducts may route the conditioned air and aerosol to locations proximate contents **624** contained within the space. Other configurations are also possible.

In some examples, the HVAC system **104** is set up in an installation with an air curtain **614.** The air curtain may be located proximate an ingress **622** to the conditioned space, and the air curtain may include an aerosol generator **216** and a fan **210.** In these examples the condensate **224** may be delivered from a condensate trap **220** to the air curtain **614** where the condensate is further delivered to an aerosol generator **216** within the air curtain.

The HVAC system **104** shown in FIG. **6** providing conditioning to space **602B** provides an illustration of an example where an air curtain **614** is included. In this example, the air curtain is located proximate an ingress **622** of space **602B,** and in this example, it is located above the ingress.

The air curtain **614** shown in FIG. **6** may include an aerosol generator **216** (not shown in FIG. **6**) and a fan **210** (not shown in FIG. **6**). In this example, the fan located at the air curtain may recirculate conditioned air within the space to provide a curtain of air at a given location. The depicted air curtain includes an inlet air register **626** that allows the conditioned air within the space to enter the air curtain and a supply air register **628** that directs the conditioned air back into the space, after it has been pressurized by the fan within the air curtain. In the depicted example, the air curtain provides a curtain of air directed by the supply air register proximate the ingress **622** of the space. Other examples may include more or less features and direct the air supplied by the air curtain in other ways.

The aerosol generator **216** included within the air curtain **614** may be substantially similar to the aerosol generators discussed above. For example, the aerosol generator may energize condensate **224** provided by conduit **616** to generate an aerosol with negative ions. In the example depicted in FIG. **6****,** the condensate is collected at a condensate producing component within the roof mounted air handling unit **608.** This condensate may be collected using a condensate trap, potentially one similar to condensate trap **220,** and the conduit may couple to the condensate trap to receive the condensate. In some examples, the aerosol generator may energize other fluids provided to generate the aerosol. For example, a water line may be provided to the air curtain and coupled to the aerosol generator. In some examples, the air curtain includes condensate producing components, and the condensate generated by these components is routed to the aerosol generator within the air curtain, potentially via a condensate trap and/or a conduit.

In the example depicted in FIG. **6** the aerosol is combined with the conditioned air circulating within the air curtain between the inlet air register **626** and the supply air register **628.** The aerosol combined with the conditioned air is supplied into the space via a curtain of air directed proximate the ingress of the conditioned space. In some examples, the air curtain may not recirculate conditioned air. For example, an air handling unit **202** may route conditioned air to the air curtain, potentially via a duct. In other examples, the air curtain may provide the aerosol from the aerosol generator into the space without any conditioned air. In these examples, the fan may be used to direct the aerosol into the space where it is combined with the conditioned air. In some examples, the air curtain further includes an aromatic dispenser **160** that includes some or all of the components discussed above.

In some examples, the conditioned space **602** may also include a door sensor **630,** which may provide an indication of an open-closed state of a door **630** at an ingress **622** to the conditioned space. The HVAC system **104** may also include control circuitry **146** operably coupled to the HVAC equipment **106,** the door sensor and the fan **210.** The control circuitry may also be configured to determine the open-closed state of the door using the door sensor, and control operation of the fan based on the open-close state of the door.

The example depicted in FIG. **6** shows a door **630** is located at the ingresses **622** to both spaces **602A** and **602B.** Each of these ingresses are also egresses. A door sensor **632** is also provided near each door, and the door sensor may be coupled to the control circuitry **146.** The door sensor may be any conventional device that may be used to determine an open-close state of a door, e.g., a magnetic sensor, electrical contact, optical sensor, etc.

In some examples, the control circuitry **146** may include control circuitry that determines that the open-closed state of the door **630** is a transition of the door from a closed state to an open state. This determination may be based on input from the door sensor **632.** In these examples, the control circuitry may include control circuitry that controls the operation of the fan **210** to increase an operating speed of the fan responsive to the transition. This may include increasing the speed of the fan from a normal speed of operation to an increased speed of operation. In some examples, in response to the control circuitry determining that the open-closed state of the door is a transition of the door from a closed state to an open state, the control circuitry may control the operation of the fan to turn the fan on in response to the transition.

In some examples, the control circuitry **146** may determine that the open-closed state of the door **630** indicates the door is in a closed state. This determination may be based on input from the door sensor **632.** In these examples, the control circuitry may include control circuitry that controls the operation of the fan **210** to continuously operate when the door is in the closed state. In some examples, control circuitry controls the fan to continuously operation when the door is in the closed state, and other conditions are also met. For example, if the conditioned space is on a moving vehicle such as a trailer or a bus, the control circuitry may operate the fan continuously when the door is in a closed state and the vehicle is moving. Other additional conditions may also be used to control the operation of the fan.

In some examples, the HVAC system **104** is configured to provide conditioned air to contents **624** within a conditioned space **602.** In these examples, the control circuitry **146** may determine a type of the content within the conditioned space. The control circuitry may further control a rate at which the aerosol is generated at the aerosol generator **216** based on the type of the contents.

The depicted examples in FIG. **6** show two separate conditioned space **(602A & 602B),** each of which include contents **624.** The type of contexts may vary. For example, the type of contents within the spaces may be plants, perishable foods, electrical components, chemicals, medicines, vaccines, or other types of contents. The HVAC system may provide conditioning and/or disinfecting based on the type of contents within the space.

In some examples, the control circuitry **146** may determine the type of contents **624** within the conditioned space **602.** This may include determining the type of some or all of the contents within the conditioned space, potentially focusing on the contents that are the most sensitive to environmental conditions and/or contaminates. The control circuitry may determine the type of contents within the conditioned space based on various methods. For example, the control circuitry may determine the type of contents by receiving a user input with this information. In some examples, the control circuitry is coupled to sensors **634** which may allow it to determine the type of contents. This includes using a radio frequency identification (RFID) reader as a sensor to scan an RFID code associated with the contents as they are loaded into the conditioned space, allowing the control circuitry to determine the contents of the space based on the indication from the RFID. Barcode scanning and other similar devices may also be used by the control circuitry in a similar manner. In other examples, the control circuitry may be coupled to optical sensors which visually determine the type of contents. Other sensors may monitor the conditions of the space to determine the contents of the space. For example, an oxygen (O2) sensor may detect a high level of O2 indicating the contents include a living plant. Or other environmental sensors, e.g., temperature, humidity, occupancy, etc., may be used to provide an indication of the type of contents.

In some examples, the control circuitry **146** controls the rate at which the aerosol is generated at the aerosol generator **216** based on the type of the content **624.** For example, the control circuitry may increase the rate at which the aerosol is generated if the type of contents within the conditioned space are particularly sensitive to contaminants. For example, plants and/or perishable foods may require certain levels of disinfect within the conditioned space, and the control circuitry may set a higher rate of aerosol to be generated when these types of contents are determined to be within the conditioned space. In some examples, the control circuitry may increase the rate of aerosol being generated once it is determined a certain type of contents is within the conditioned space. For example, the control circuitry may determine either a plant or a perishable food enters the conditioned space, and the control circuitry may increase the rate at which aerosol is being generated based on this determination. In other examples, the control circuitry may determine the contents within the space require less aerosol. For example, the contents may be certain types of cargo that are less sensitive to environmental conditions such as building materials. In these examples the control circuitry may set the rate at which aerosol is generated to a lower level or potentially off. The control circuitry may also set and adjust the rate at which aerosol is generated based on other factors or settings.

In some examples, the conditioned space **602** is divided into compartments (e.g., **602A** and **602B**) to which the conditioned air is provided. In these examples, the fan **210** may be operated to move portions of the aerosol toward respective ones of the compartments. In these examples, the HVAC system **104** may also include supply air ductwork that routes the aerosol into each of the compartments within the conditioned space. In some examples the aerosol is combined with the conditioned air once it is routed into each compartment. In some examples the aerosol is combined with the conditioned air prior to entering the compartments, potentially being combined in either the return air path or supply air path of an air handling unit **202.**

In some examples, the conditioned space **602** is divided into compartments (e.g., **602A** and **602B**) to which the conditioned air is provided, and the HVAC system **104** is set up in an installation with aerosol generators **216** and fans **210** for respective ones of the compartments. In these examples, the condensate **224** may be delivered from the condensate trap **220** to the aerosol generators at which the condensate is energized to generate respective aerosols including negative ions. In these examples, the fans may also be operable to move the respective aerosols toward respective ones of the compartments.

In these examples, condensate **224** may be produced in one or more condensate producing components within the HVAC system **104.** The condensate may be collected in condensate trap(s) and routed via conduits **222** to one or more aerosol generators **216.** For example, a portion of the condensate may be routed to a first aerosol generator associated with one of the compartments of the condition space, and another portion of the condensate may be routed to a second aerosol generator associated with the other compartment of the condition space. Each of the first and second aerosol generators may generate aerosol, e.g., a first and a second aerosol respectively, from the condensate provided. The HVAC system may also include a fan **210** associated with each aerosol generator, for example, a first fan may be associated with the first aerosol. The HVAC system may also include a second fan associated with the second aerosol generator. In some examples, one or more of the aerosol generators is associated with an air handling units and the fan is a part of the air handling unit. These fans may move the aerosol from each of the respective aerosol generators into the compartments (**602A** and **602B**) of the conditioned spaces 602. In some examples, the control circuitry **146** controls these aerosol generators and their respective fans as described above. In some of the examples, the control circuitry controls each aerosol generator and corresponding fan for each compartment independently, and in some examples, these components are controlled jointly. In some examples, the condensate is routed to more than two aerosol generators associated with compartments and/or conditioned spaces that operate in a similar manner.

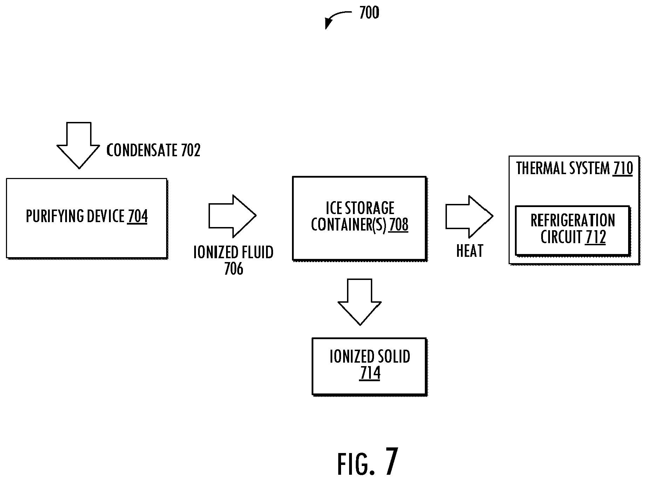

FIG. **7** shows an example diagram of a purifying system **700** that may utilize some or all of the components discussed above. The purifying system may receive condensate **702** or other fluids in the same or a similar manner as discussed in the above examples, including receiving condensate from the HVAC system **104** with HVAC equipment **106.** The purifying system may also include a purifying device **704.** The purifying device may be substantially the same as the air purifying device **102** discussed above, including all of the same components for water shearing and/or field charging. In addition, the purifying device **704** may produce an ionized fluid 706 in the same or a similar manner as discussed in the above examples. In some examples, the ionized fluid is aerosol with negative ions as described in the above examples. In some examples, the ionized fluid is a liquid ionized fluid that is ionized using the same processes discussed above for the aerosol. In these examples, the liquid ionized fluid may be the fluid remaining after the various ionizing processes, e.g., the shearing and/or field charging, that does not result in the aerosol.

The purifying system **700** may also include ice storage container(s) **708** that may receive the ionized fluid **706.** The ice storage container(s) may be thermally coupled to a thermal system **710** such as a refrigeration circuit **712,** and the thermal system may freeze the combined aerosol and fluid by removing heat. This freezing process may result in an ionized solid **714** being formed from the ionized fluid.

In some examples, the purifying system **700** uses the HVAC system **104** with HVAC equipment **106** and the purifying device **704** to form a disinfectant. This disinfectant may be formed using several of the devices and processes discussed above. For example, the purifying device **704** may include an aerosol generator **216,** which may produce the ionized fluid in both aerosol and ionized liquid form. In these examples, condensate **224** from a condensate trap **220** may be delivered to an aerosol generator **216** via a conduit **222.** The condensate may be energized at the aerosol generator to generate ionized fluid **706,** which may be aerosol and/or a liquid fluid that includes negative ions. The purifying system may also include one or more ice storage containers **708** within which the ionized fluid **706** may be contained. One or more ice storage containers **708** may be thermally coupled to a refrigerant circuit **712** usable to remove heat from the combined aerosol and fluid within the one or more ice storage containers, and thereby freeze the ionized fluid to form an ionized solid **714** that is usable as a disinfectant.

In some examples, the purifying system **700** further includes a receptacle (not shown) for storing the ionized solid with perishable food to preserve perishable food. This configuration may cool the space within the receptacle and also disinfect the perishable food and surrounding environment as the ionized solid melts.

The purifying system **700** may include multiple approaches to preserving perishable goods, such as food. For example, the ionized solid may be crushed, which may facilitate a phase change to a liquid, and the perishable goods may be stored with the crushed and/or liquefied ionized solid. In some examples, the ionized fluid from the purifying device **704** is routed directly to the perishable goods, this including routing the ionized liquid fluid directly to these goods, potentially via a conduit or other mechanism.

In some of these embodiments, the refrigeration circuit **712** may include a heat exchanger **802** and an ice storage tank **804.** The ice storage tank may contain one or more ice storage containers **708** loaded within the ice storage tank, and from which the one or more ice storage containers may be unloaded. In these examples, the refrigeration circuit may circulate a refrigerant fluid to transfer thermal energy between the one or more ice storage containers loaded within the ice storage tank and the heat exchanger.

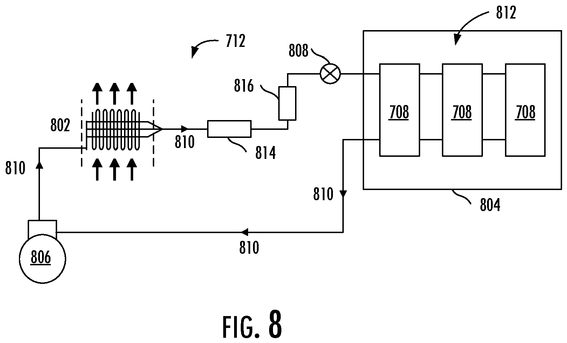

FIG. **8** shows an illustration an example refrigeration circuit **712** that may be used with the purifying system **700.** In this depicted example, the refrigeration circuit **712** includes a compressor **806,** a first heat exchanger **802,** a metering device **808,** and an ice storage tank **804.** The compressor is used to circulate a refrigerant fluid **810** between the various components, transferring thermal energy in a desired manner. In the depicted embodiment, the refrigerant fluid is circulated from the compressor to the first heat exchanger where heat may be transferred from the refrigerant fluid out of the system. In some examples, the refrigerant fluid condenses at the first heat exchanger. The refrigerant fluid is then circulated to the metering device, which reduces the pressure of the refrigerant fluid potentially lowering the temperature. Any standard metering device may be used, such as an electronic expansion valve (EEV), a thermostatic expansion valve, a capillary tube assembly, and/or any other suitable metering device. The refrigerant fluid is then circulated to the ice storage tank **804** where the refrigerant fluid removes heat from the ice storage tank, lowering the temperature of the contents within the ice storage tank, potentially freezing the contents.

In some of these examples, the ice storage tank **804** may include one or more ice storage containers **708** loaded within the ice storage tank. The ice storage tank may also include a brine tank **812** that includes a brine solution and serves as a thermal reservoir for the ice storage tank. The brine solution may have a lower freezing point to allow brine solution to remain a liquid when other components within the ice storage tank freeze, e.g., the contents of the ice storage containers. In some examples the brine solution includes glycol, and in some examples, the brine solution is a mixture of water and glycol.

As shown in FIG. **8****,** the refrigeration circuit **712** may also include additional components. In the depicted example, a receiver **814** and a drier **816** are also included as part of the refrigeration circuit.

In some examples, the HVAC equipment **104** includes a transport refrigeration unit that includes the refrigeration circuit **712** with a heat exchanger **802,** the condensate being produced at the heat exchanger and routed to the ice storage containers **708.** In these examples, the condensate collected may be considered the condensate **702** that is used to generated by the purifying device **704** to generate the ionized fluid **706.** In these examples, a condensate trap (not shown in FIG. **8**), potentially similar to the same as condensate trap **220,** is used to collect the condensate. A conduit, similar to the same as conduit **222,** may be used to route the condensate to the purifying device, which is then used to generate the ionized fluid that is routed to the ice storage container(s) **708.**

In some examples, additional fluids may be routed to the purifying device **704.** This may be because the condensate generated and/or routed to the purifying device is insufficient to provide the desired quantity of ionized fluid **706.** In these examples, additional fluid sources, including water lines or other water sources, may provide fluid to the purifying device to generate the ionized fluid. In some examples, the additional fluids undergo an additional filtration or disinfecting process before arriving at the purifying device. Other examples may use other fluids or other forms of routing the fluid to purifying device.

In some examples where the fluid includes condensate **224,** the condensate may be filtered and disinfected using the processes described above. In these examples, the aerosol may be combined with the condensate as filtered and disinfected.

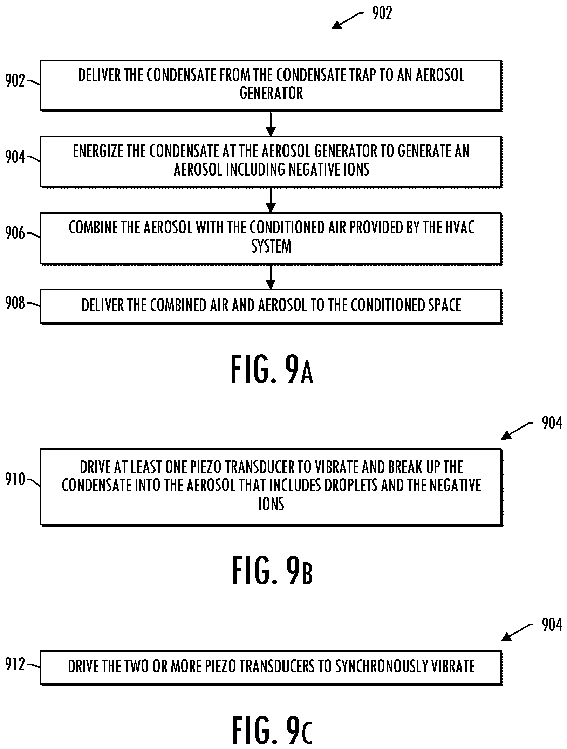

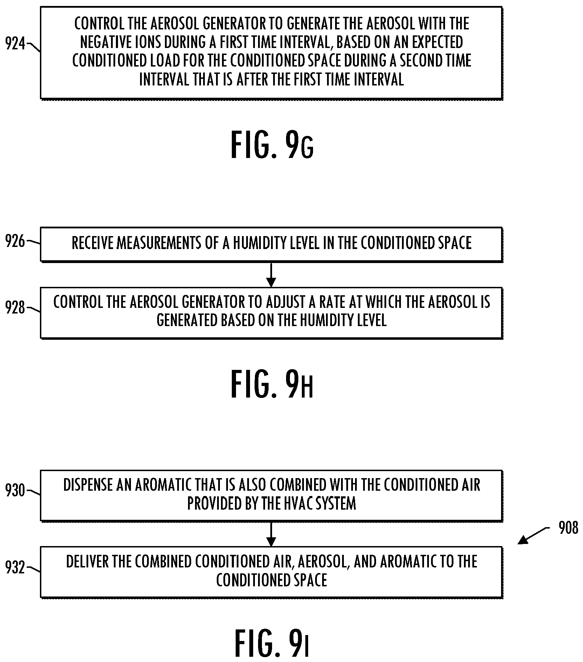

FIGS. **9A** - **91** are flowcharts illustrating various steps in a method **900** of disinfecting a conditioned space to which heating ventilation, and air conditioning (HVAC) equipment **106** of an HVAC system **104** is configured to provide conditioned air, the HVAC system including a condensate trap **220** configured to collect condensate **224** produced during operation, according to various example implementations of the present disclosure. The method includes delivering the condensate from the condensate trap to an aerosol generator **216,** as shown at block **902** of FIG. **9A****.** The method includes energizing the condensate at the aerosol generator to generate an aerosol including negative ions, as shown at block **904.** The method includes combining the aerosol with the conditioned air provided by the HVAC system, as shown at block **906.** And the method includes delivering the combined air and aerosol to the conditioned space, as shown at block **908.**

In some examples, energizing the condensate at block **904** includes driving at least one piezo transducer to vibrate and break up the condensate into the aerosol that includes droplets and the negative ions, as shown at block **910** of FIG. **9B****.**

In some examples, the aerosol is generated such that the droplets are liquid nanoparticle droplets. In some examples, the aerosol is generated such that the droplets are liquid microparticles.

In some examples, the at least one piezo transducer is two or more piezo transducers, and energizing the condensate at block **904** further includes driving the two or more piezo transducers to synchronously vibrate, as shown at block **912** of FIG. **9C****.**

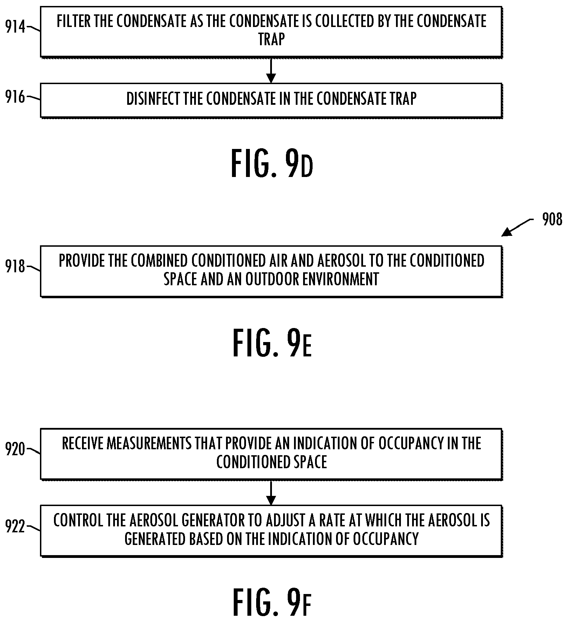

In some examples, the method **900** further includes filtering the condensate as the condensate is collected by the condensate trap, and disinfecting the condensate in the condensate trap, as shown at blocks **916** and **914** of FIG. **9D****.**

In some examples, the delivering the combined air and aerosol at block **908** further includes providing the combined conditioned air and aerosol to the conditioned space and an outdoor environment, as shown at block **918** of FIG. **9E****.**

In some examples, the method **900** further includes receiving measurements that provide an indication of occupancy in the conditioned space, as shown at block **920** of FIG. **9F****.** And the method includes controlling the aerosol generator to adjust a rate at which the aerosol is generated based on the indication of occupancy, as shown at block **922.**

In some examples, the method **900** further includes controlling the aerosol generator to generate the aerosol with the negative ions during a first time interval, based on an expected conditioned load for the conditioned space during a second time interval that is after the first time interval, as shown at block **924** of FIG. **9G****.**

In some examples, the method **900** further includes receiving measurements of a humidity level in the conditioned space, as shown at block **926** of FIG. **9H****.** And the method includes controlling the aerosol generator to adjust a rate at which the aerosol is generated based on the humidity level, as shown at block **928.**

In some examples, the method **900** further includes dispensing an aromatic that is also combined with the conditioned air provided by the HVAC system, as shown at block **930** of FIG. **91.** In some of these examples, delivering the combined air and aerosol at block **908** includes delivering the combined conditioned air, aerosol, and aromatic to the conditioned space, as shown at block **932.**

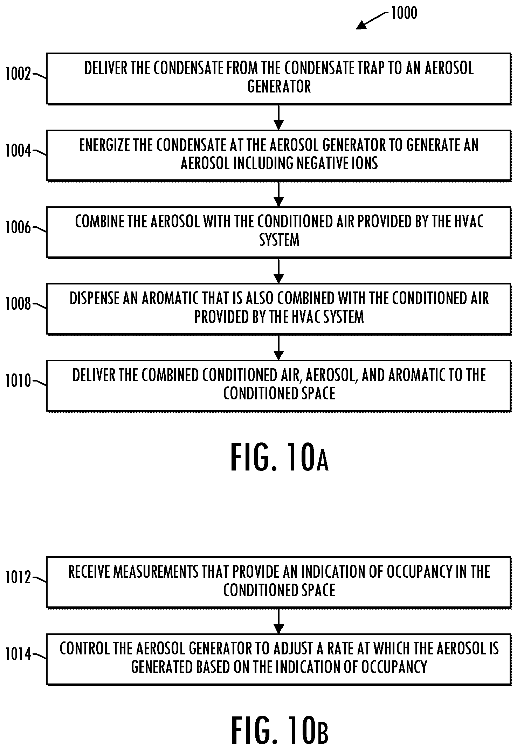

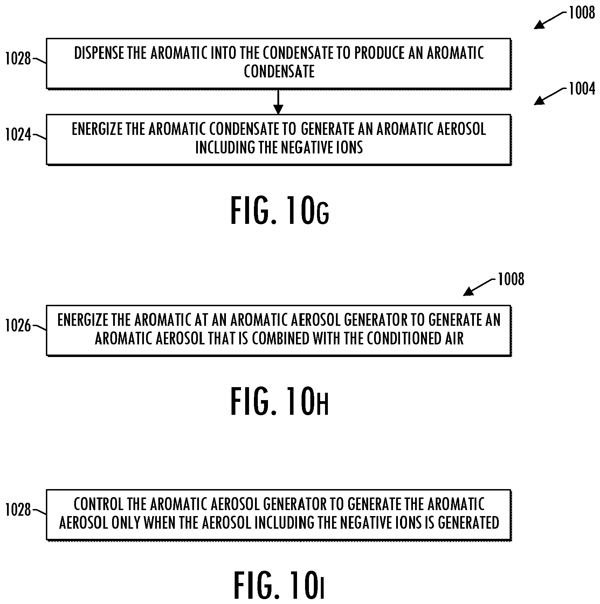

FIGS. **10A** - **101** are flowcharts illustrating various steps in a method **1000** of disinfecting a conditioned space to which heating ventilation, and air conditioning (HVAC) equipment **106** of an HVAC system **104** is configured to provide conditioned air, the HVAC system including a condensate trap**220** configured to collect condensate **224** produced during operation, according to various example implementations of the present disclosure. The method includes delivering the condensate from the condensate trap to an aerosol generator, as shown at block **1002** of FIG. **10A****.** The method includes energizing the condensate at the aerosol generator to generate an aerosol including negative ions, as shown at block **1004.** The method includes combining the aerosol with the conditioned air provided by the HVAC system, as shown at block **1006.** The method includes dispensing an aromatic **502** that is also combined with the conditioned air provided by the HVAC system, as shown at block **1008.** And the method includes delivering the combined conditioned air, aerosol, and aromatic to the conditioned space, as shown at block **1010.**

In some examples, the method **1000** further includes receiving measurements that provide an indication of occupancy in the conditioned space, as shown at block **1012** of FIG. **10B****.** In some of these examples, the method also includes controlling the aerosol generator to adjust a rate at which the aerosol is generated based on the indication of occupancy, as shown at block **1014.**

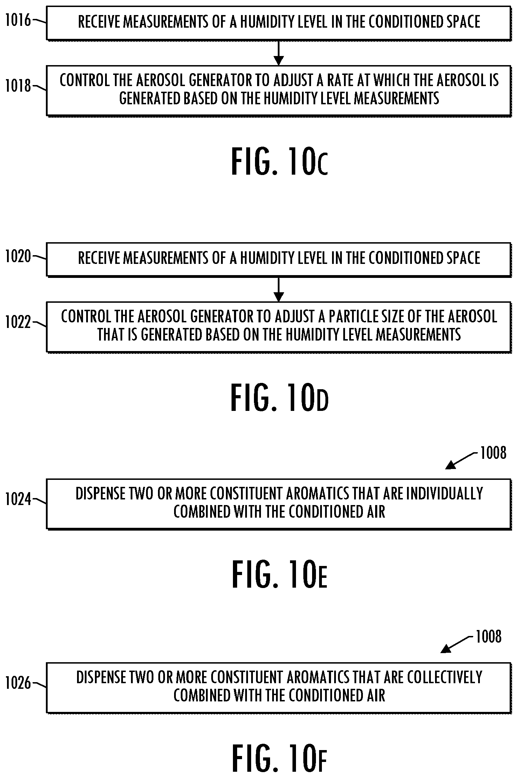

In some examples, the method **1000** further includes receiving measurements of a humidity level in the conditioned space, as shown at block **1016** of FIG. **10C****.** In some of these examples, the method also includes controlling the aerosol generator to adjust a rate at which the aerosol is generated based on the humidity level measurements, as shown at block **1018.**

In some examples, the method **1000** further includes receiving measurements of a humidity level in the conditioned space, as shown at block **1020** of FIG. **10D****.** In some of these examples, the method also includes controlling the aerosol generator to adjust a particle size of the aerosol that is generated based on the humidity level measurements, as shown at block **1022.**

In some examples, dispensing the aromatic at block **1008** includes dispensing two or more constituent aromatics that are individually combined with the conditioned air, as shown at block **1024** of FIG. **10E****.**

In some examples, dispensing the aromatic at block **1008** includes dispensing two or more constituent aromatics that are collectively combined with the conditioned air, as shown at block **1026** of FIG. **10F****.**

In some examples, the aromatic is a water soluble tablet.

In some examples, the dispensing the aromatic at block **1008** includes dispensing the aromatic into the condensate to produce an aromatic condensate, as shown at block **1028** of FIG. **10G****.** In some of these examples, energizing the condensate at block **1004** includes energizing the aromatic condensate to generate an aromatic aerosol including the negative ions, as shown at block **1024.**

In some examples, the dispensing the aromatic at block **1008** further includes energizing the aromatic at an aromatic aerosol generator to generate an aromatic aerosol that is combined with the conditioned air, as shown at block **1026** of FIG. **10H****.**

In some examples, the method **1000** further includes controlling the aromatic aerosol generator to generate the aromatic aerosol only when the aerosol including the negative ions is generated, as shown at block **1028** of FIG. **101.**

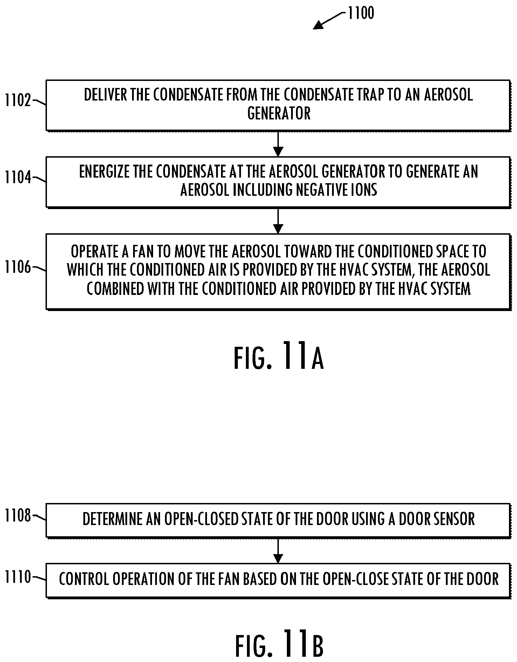

FIGS. **11A - 11G** are flowcharts illustrating various steps in a method **1100** of disinfecting a conditioned space to which a heating ventilation, and air conditioning (HVAC) system with HVAC equipment is configured to provide conditioned air, the HVAC system including a condensate trap configured to collect condensate produced during operation, according to various example implementations of the present disclosure. The method includes delivering the condensate from the condensate trap to an aerosol generator, as shown at block **1102** of FIG. **11A****.** The method includes energizing the condensate at the aerosol generator to generate an aerosol including negative ions, as shown at block **1104.** And the method includes operating a fan to move the aerosol toward the conditioned space to which the conditioned air is provided by the HVAC system, the aerosol combined with the conditioned air provided by the HVAC system, as shown at block **1106.**

In some examples, the HVAC system is set up in an installation with a duct that defines a pathway to a location proximate an ingress to the conditioned space, and the fan is operated at block **1106** to move the aerosol through the duct using a fan, toward the location and thereby the ingress to the conditioned space.

In some examples, the HVAC system is set up in an installation with an air curtain located proximate an ingress to the conditioned space, and the air curtain includes the aerosol generator and the fan. In some of these examples, the condensate is delivered at block **1102** from the condensate trap to the air curtain and thereby the aerosol generator.

In some examples, the conditioned space further includes a door at an ingress to the conditioned space. The method further includes determining an open-closed state of the door using a door sensor, as shown at block **1108** of FIG. 11B. And the method includes controlling operation of the fan based on the open-close state of the door, as shown at block **1110.**

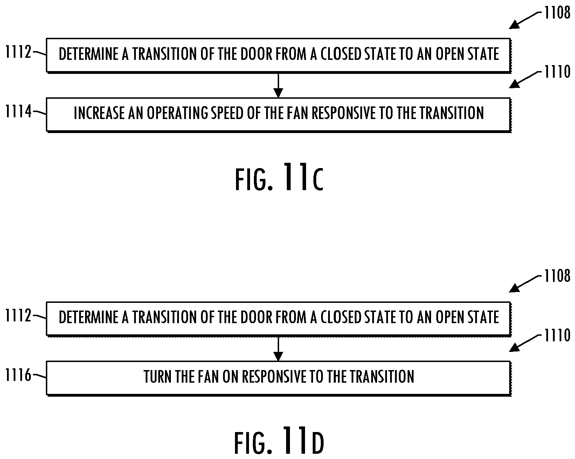

In some examples, determining the open-closed state of the door at block 1108 includes determining a transition of the door from a closed state to an open state, as shown at block **1112** of FIG. **11C****.** In some of these examples, controlling the operation of the fan at block **1110** includes increasing an operating speed of the fan responsive to the transition, as shown at block **1114.**

In some examples, determining the open-closed state of the door at block **1108** includes determining a transition of the door from a closed state to an open state, as shown at block **1112** of FIG. **11D****.** In some of these examples, controlling the operation of the fan at block **1110** includes turning the fan on responsive to the transition, as shown at block **1116.**

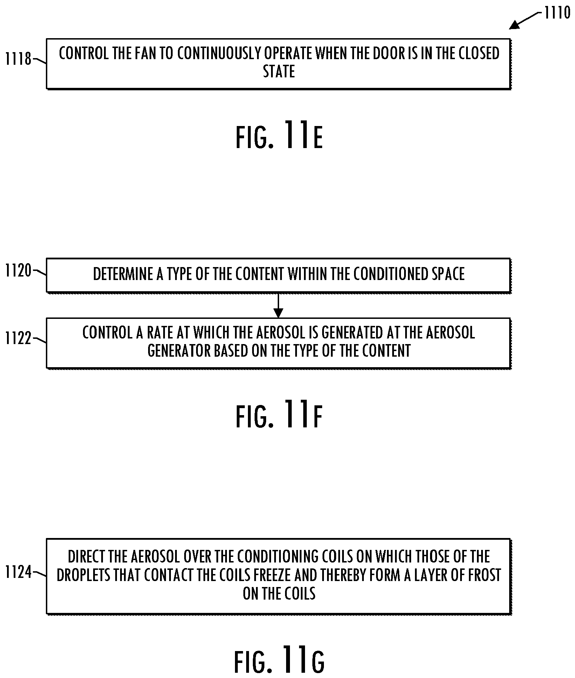

In some examples, the open-closed state of the door indicates the door is in a closed state. And in some of these examples, controlling the operation of the fan at block **1110** includes controlling the fan to continuously operate when the door is in the closed state, as shown at block **1118** of FIG. **11E****.**

In some examples, the HVAC system is configured to provide the conditioned air to content within the conditioned space. In some of these examples, the method **1100** further includes determining a type of the content within the conditioned space, as shown at block **1120** of FIG. **11F****.** And the method includes controlling a rate at which the aerosol is generated at the aerosol generator based on the type of the content, as shown at block **1122.**

In some examples, the type of the content is determined as a plant or a perishable food.

In some examples, the conditioned space is divided into compartments to which the conditioned air is provided, and the fan is operated at block **1106** to move portions of the aerosol toward respective ones of the compartments.

In some examples, the conditioned space is divided into compartments to which the conditioned air is provided, and the HVAC system is set up in an installation with aerosol generators and fans for respective ones of the compartments. In some of these examples, the condensate is delivered at block **1102** from the condensate trap to the aerosol generators at which the condensate is energized at block **1104** to generate respective aerosols including negative ions. And the fans are operated at block **1106** to move the respective aerosols toward respective ones of the compartments.

In some examples, the HVAC equipment includes conditioning coils, and the aerosol includes droplets of the condensate. In some of these examples, the method **1100** further includes directing the aerosol over the conditioning coils on which those of the droplets that contact the coils freeze and thereby form a layer of frost on the coils, as shown at block **1124** of FIG. **11G****.**

In some examples, the HVAC equipment includes a transport refrigeration unit configured to provide the conditioned air to the conditioned space.

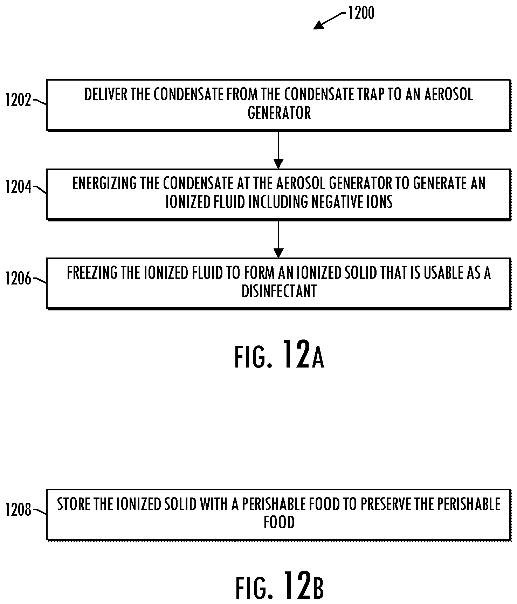

FIGS. **12A - 12E** are flowcharts illustrating various steps in a method **1200** of forming a disinfectant from condensate produced by and during operation of a heating ventilation, and air conditioning (HVAC) system with HVAC equipment configured to provide conditioned air, the HVAC system including a condensate trap configured to collect the condensate, according to various example implementations of the present disclosure. The method includes delivering the condensate from the condensate trap to an aerosol generator, as shown at block **1202** of FIG. **12A****.** The method includes energizing the condensate at the aerosol generator to generate an ionized fluid including negative ions, as shown at block **1204.** And the method includes freezing the ionized fluid to form an ionized solid that is usable as a disinfectant, as shown at block **1206.**

In some examples, the method **1200** further includes storing the ionized solid with a perishable food to preserve the perishable food, as shown at block **1208** of FIG. **12B****.**

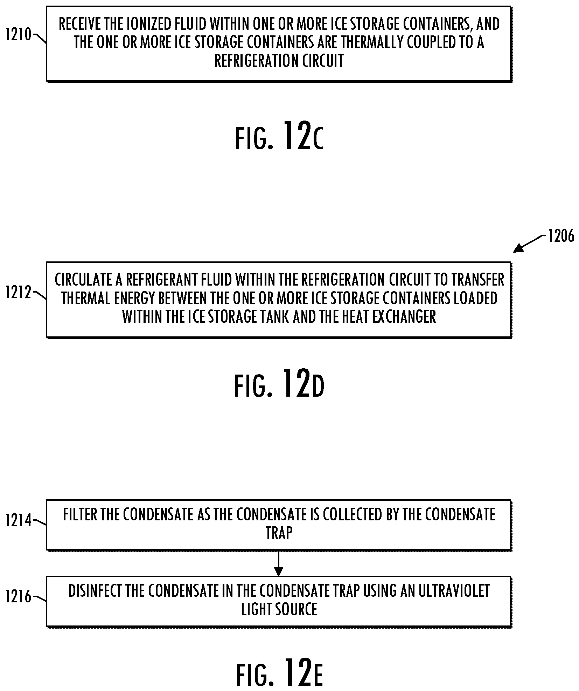

In some examples, the method **1200** further includes receiving the ionized fluid within one or more ice storage containers, and the one or more ice storage containers are thermally coupled to a refrigeration circuit, as shown at block **1210** of FIG. **12C****.** In some of these examples, the ionized fluid is frozen at block **1206** using the refrigeration circuit to remove heat from the combined aerosol and fluid within the one or more ice storage containers.

In some examples, the refrigeration circuit includes a heat exchanger, and an ice storage tank within which the one or more ice storage containers are loaded, and from which the one or more ice storage containers are unloadable. In some of these examples, freezing the ionized fluid at block **1206** includes circulating a refrigerant fluid within the refrigeration circuit to transfer thermal energy between the one or more ice storage containers loaded within the ice storage tank and the heat exchanger, as shown at block **1212** of FIG. **12D****.**

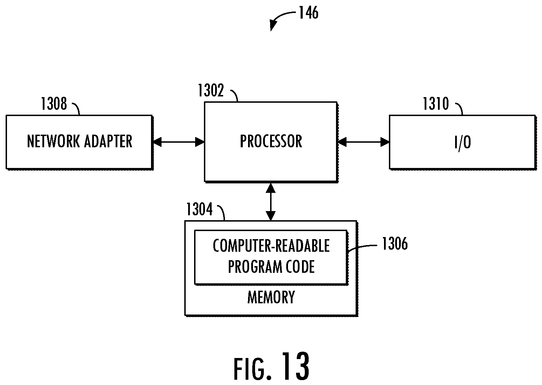

In some examples, the fluid comprises the condensate, and the method **1200** further includes filtering the condensate as the condensate is collected by the condensate trap, as shown at block **1214** of FIG. **12E****.** The method includes disinfecting the condensate in the condensate trap using an ultraviolet light source, as shown at block **1216.** And in some of these examples, the aerosol is combined with the condensate as filtered and disinfected.FIG. **13** illustrates the control circuitry **146** according to some example implementations of the present disclosure. The control circuitry may include one or more of each of a number of components such as, for example, a processor **1302** connected to a memory **1304.** The processor is generally any piece of computer hardware capable of processing information such as, for example, data, computer programs and/or other suitable electronic information. The processor includes one or more electronic circuits some of which may be packaged as an integrated circuit or multiple interconnected integrated circuits (an integrated circuit is at times more commonly referred to as a "chip"). The processor **1302** may be a number of processors, a multi-core processor, or some other type of processor, depending on the particular implementation.

The processor **1302** may be configured to execute computer programs such as computer-readable program code **1306,** which may be stored onboard the processor or otherwise stored in the memory **1304.** In some examples, the processor may be embodied as or otherwise include one or more ASICs, FPGAs or the like. Thus, although the processor may be capable of executing a computer program to perform one or more functions, the processor of various examples may be capable of performing one or more functions without the aid of a computer program.

The memory **1304** is generally any piece of computer hardware capable of storing information, such as, for example, data, computer-readable program code **1306** or other computer programs, and/or other suitable information either on a temporary basis and/or a permanent basis. The memory may include volatile memory such as random access memory (RAM), and/or non-volatile memory such as a hard drive, flash memory or the like. In various instances, the memory may be referred to as a computer-readable storage medium, which is a non-transitory device capable of storing information. In some examples, then, the computer-readable storage medium is non-transitory and has computer-readable program code stored therein, which, in response to execution by the processor **1302,** causes the control circuitry **146** to perform various operations as described herein, some of which may in turn cause the air purifying system **100** to perform various operations.

In addition to the memory **1304,** the processor **1302** may also be connected to one or more peripherals such as a network adapter **1308,** one or more input/output (I/O) devices **1310,** or the like. The network adapter is a hardware component configured to connect the control circuitry **146** to a computer network to enable the control circuitry to transmit and/or receive information via the computer network. The I/O devices may include one or more input devices capable of receiving data or instructions for the control circuitry, and/or one or more output devices capable of providing an output from the control circuitry. Examples of suitable input devices include a keyboard, keypad or the like, and examples of suitable output devices include a display device such as a one or more light-emitting diodes (LEDs), a LED display, a liquid crystal display (LCD), or the like.

As explained above and reiterated below, the present disclosure includes, without limitation, the following example implementations.

Clause 1. An air purifying system comprising: a heating, ventilation and air conditioning (HVAC) system with HVAC equipment configured to provide conditioned air to a conditioned space, the HVAC system including a condensate trap configured to collect condensate produced during operation; and an air purifying device configured to energize the condensate from the condensate trap to generate an aerosol including negative ions, the aerosol being combinable with the conditioned air provided by the HVAC system for delivery to the conditioned space.

Clause 2. The air purifying system of clause 1, wherein the HVAC system is set up in an installation that defines an air supply path, and wherein the air purifying device includes an aerosol generator located in the air supply path and configured to energize the condensate.

Clause 3. The air purifying system of clause 2, wherein the HVAC equipment includes a heat exchanger coil located in the air supply path, wherein the heat exchanger coil is arranged downstream from the aerosol generator in the air supply path.

Clause 4. The air purifying system of any of clauses 1 to 3, further comprising: a particle filter configured to filter the condensate as the condensate is collected by the condensate trap; and an ultraviolet light source configured to disinfect the condensate in the condensate trap.

Clause 5. The air purifying system of any of clauses 1 to 4, wherein the HVAC system is configured to provide the conditioned air combined with the aerosol including the negative air ions, to the conditioned space and an outdoor environment.

Clause 6. The air purifying system of any of clauses 1 to 5, wherein the HVAC system further includes control circuitry operably coupled to the HVAC equipment and the air purifying device, and wherein the HVAC equipment further includes a sensor configured to provide an indication of occupancy in the conditioned space, and the control circuitry is configured to control the air purifying device to adjust a rate at which the aerosol is generated by the air purifying device based on the indication of occupancy.

Clause 7. The air purifying system of any of clauses 1 to 6, wherein the HVAC system further includes control circuitry operably coupled to the HVAC equipment and the air purifying device, and the control circuitry is configured to control the air purifying device to generate the aerosol with the negative ions during a first time interval, based on an expected conditioned load for the conditioned space during a second time interval that is after the first time interval.

Clause 8. The air purifying system of any of clauses 1 to 7, wherein the HVAC system further includes control circuitry operably coupled to the HVAC equipment and the air purifying device, and wherein the HVAC equipment further includes a humidity sensor configured to provide measurements of a humidity level in the conditioned space, and the control circuitry is configured to adjust a rate at which aerosol is generated by the air purifying device based on the measurements of the humidity level.

Clause 9. The air purifying system of any of clauses 1 to 8, further comprising an aromatic dispenser configured to dispense an aromatic that is also combinable with the conditioned air provided by the HVAC system for delivery to the conditioned space, the aromatic combinable with the conditioned air before the conditioned air reaches the conditioned space.

Clause 10. An air purifying system comprising: a heating, ventilation and air conditioning (HVAC) system with HVAC equipment configured to provide conditioned air to a conditioned space, the HVAC system including a condensate trap configured to collect condensate produced during operation; an air purifying device configured to energize the condensate from the condensate trap to generate an aerosol including negative ions, the aerosol being combinable with the conditioned air provided by the HVAC system for delivery to the conditioned space; and an aromatic dispenser configured to dispense an aromatic that is also combinable with the conditioned air provided by the HVAC system for delivery to the conditioned space, the aromatic combinable with the conditioned air before the conditioned air reaches the conditioned space.

Clause 11. The air purifying system of clause 10, wherein the HVAC system is set up in an installation that defines an air supply path, and wherein the air purifying device includes an aerosol generator located in the air supply path and configured to energize the condensate.

Clause 12. The air purifying system of clause 10 or clause 11, wherein the HVAC equipment includes a sensor configured to provide an indication of occupancy in the conditioned space, and the HVAC system further includes control circuitry operably coupled to the HVAC equipment and the air purifying device, and wherein the control circuitry is configured to adjust a rate at which aerosol is generated by the air purifying device based on the indication of occupancy.

Clause 13. The air purifying system of any of clauses 10 to 12, wherein the HVAC equipment further includes a humidity sensor configured to provide measurements of a humidity level in the conditioned space, and the HVAC system further includes control circuitry operably coupled to the HVAC equipment and the air purifying device, and wherein the control circuitry is configured to adjust a rate at which aerosol is generated by the air purifying device based on the measurements of the humidity level.

Clause 14. The air purifying system of any of clauses 10 to 13, wherein the HVAC equipment further includes a humidity sensor configured to provide measurements of a humidity level in the conditioned space, and the HVAC system further includes control circuitry operably coupled to the HVAC equipment and the air purifying device, and wherein the control circuitry is configured to adjust a particle size of the aerosol that is generated by the air purifying device based on the measurements of the humidity level.

Clause 15. The air purifying system of any of clauses 10 to 14, wherein the aromatic dispenser is configured to dispense two or more constituent aromatics that are individually combinable with the conditioned air.

Clause 16. The air purifying system of any of clauses 10 to 15, wherein the aromatic dispenser is configured to dispense two or more constituent aromatics that are collectively combinable with the conditioned air.

Clause 17. The air purifying system of any of clauses 10 to 16, wherein the aromatic is a water soluble tablet.

Clause 18. The air purifying system of any of clauses 10 to 17, wherein the aromatic dispenser is configured to dispense the aromatic into the condensate using a dispenser to produce an aromatic condensate, and the air purifying device is configured to energize the aromatic condensate to generate an aromatic aerosol including the negative ions.

Clause 19. The air purifying system of any of clauses 10 to 18, wherein the aromatic dispenser includes an aromatic aerosol generator configured to energize the aromatic to generate an aromatic aerosol that is combinable with the conditioned air.

Clause 20. The air purifying system of clause 19, further comprising control circuitry configured to control the air purifying device to generate the aerosol including the negative ions, the control circuitry also configured to control the aromatic aerosol generator to generate the aromatic aerosol only when the aerosol including the negative ions is generated.

Clause 21. An air purifying device for a heating, ventilation and air conditioning (HVAC) system operable to provide conditioned air to a conditioned space, the HVAC system including a condensate trap configured to collect condensate produced during operation of the HVAC system, the air purifying device comprising: an aerosol generator; and a conduit operably coupleable to and between the condensate trap and the aerosol generator, the conduit configured to deliver the condensate from the condensate trap to the aerosol generator, the aerosol generator configured to energize the condensate to generate an aerosol including negative ions combinable with the conditioned air provided by the HVAC system for delivery to the conditioned space.

Clause 22. The air purifying device of clause 21, wherein the aerosol generator comprises: at least one piezo transducer; and a driver circuit configured to drive the at least one piezo transducer to vibrate and break up the condensate into the aerosol that includes droplets and the negative ions.

Clause 23. The air purifying device of clause 22, wherein the aerosol generator is configured such that the droplets are liquid nanoparticles.

Clause 24. The air purifying device of clause 22 or clause 23, wherein the at least one piezo transducer is two or more piezo transducers, and the driver circuit is configured to drive the two or more piezo transducers to synchronously vibrate.

Clause 25. The air purifying device of any of clauses 21 to 24, wherein the aerosol generator is configured to generate the aerosol with the negative ions under control from the HVAC system, and based on an indication of occupancy in the conditioned space.

Clause 26. The air purifying device of any of clauses 21 to 25, further comprising an aromatic dispenser configured to dispense an aromatic that is also combinable with the conditioned air provided by the HVAC system for delivery to the conditioned space, the aromatic combinable with the conditioned air before the conditioned air reaches the conditioned space.

Clause 27. A method of disinfecting a conditioned space to which heating, ventilation, and air conditioning (HVAC) equipment of an HVAC system is configured to provide conditioned air, the HVAC system including a condensate trap configured to collect condensate produced during operation, the method comprising: delivering the condensate from the condensate trap to an aerosol generator; energizing the condensate at the aerosol generator to generate an aerosol including negative ions; combining the aerosol with the conditioned air provided by the HVAC system; and delivering the combined air and aerosol to the conditioned space.

Clause 28. The method of clause 27, wherein energizing the condensate includes driving at least one piezo transducer to vibrate and break up the condensate into the aerosol that includes droplets and the negative ions.

Clause 29. The method of clause 28, wherein the aerosol is generated such that the droplets are liquid nanoparticle droplets.

Clause 30. The method of clause 28 or clause 29, wherein the aerosol is generated such that the droplets are liquid microparticles.

Clause 31. The method of any of clauses 28 to 30, wherein the at least one piezo transducer is two or more piezo transducers, and energizing the condensate further includes driving the two or more piezo transducers to synchronously vibrate.

Clause 32. The method of any of clauses 27 to 31, further comprising filtering the condensate as the condensate is collected by the condensate trap, and disinfecting the condensate in the condensate trap.

Clause 33. The method of any of clauses 27 to 32, wherein the delivering the combined air and aerosol further includes providing the combined conditioned air and aerosol to the conditioned space and an outdoor environment.

Clause 34. The method of any of clauses 27 to 33, further comprising: receiving measurements that provide an indication of occupancy in the conditioned space; and controlling the aerosol generator to adjust a rate at which the aerosol is generated based on the indication of occupancy.

Clause 35. The method of any of clauses 27 to 34, further comprising: controlling the aerosol generator to generate the aerosol with the negative ions during a first time interval, based on an expected conditioned load for the conditioned space during a second time interval that is after the first time interval.

Clause 36. The method of any of clauses 27 to 35, further comprising: receiving measurements of a humidity level in the conditioned space; and controlling the aerosol generator to adjust a rate at which the aerosol is generated based on the humidity level.

Clause 37. The method of any of clauses 27 to 36, further comprising dispensing an aromatic that is also combined with the conditioned air provided by the HVAC system, and wherein delivering the combined air and aerosol includes delivering the combined conditioned air, aerosol, and aromatic to the conditioned space.

Clause 38. A method of disinfecting a conditioned space to which heating, ventilation, and air conditioning (HVAC) equipment of an HVAC system is configured to provide conditioned air, the HVAC system including a condensate trap configured to collect condensate produced during operation, the method comprising: delivering the condensate from the condensate trap to an aerosol generator; energizing the condensate at the aerosol generator to generate an aerosol including negative ions; combining the aerosol with the conditioned air provided by the HVAC system; dispensing an aromatic that is also combined with the conditioned air provided by the HVAC system; and delivering the combined conditioned air, aerosol, and aromatic to the conditioned space.

Clause 39. The method of clause 38, further comprising: receiving measurements that provide an indication of occupancy in the conditioned space; and controlling the aerosol generator to adjust a rate at which the aerosol is generated based on the indication of occupancy.

Clause 40. The method of clause 38 or clause 39, further comprising: receiving measurements of a humidity level in the conditioned space; and controlling the aerosol generator to adjust a rate at which the aerosol is generated based on the humidity level measurements.

Clause 41. The method of any of clauses 38 to 40, further comprising: receiving measurements of a humidity level in the conditioned space; and controlling the aerosol generator to adjust a particle size of the aerosol that is generated based on the humidity level measurements.

Clause 42. The method of any of clauses 38 to 41, wherein dispensing the aromatic includes dispensing two or more constituent aromatics that are individually combined with the conditioned air.

Clause 43. The method of any of clauses 38 to 42, wherein dispensing the aromatic includes dispensing two or more constituent aromatics that are collectively combined with the conditioned air.

Clause 44. The method of any of clauses 38 to 43, wherein the aromatic is a water soluble tablet.

Clause 45. The method of any of clauses 38 to 44, wherein the dispensing the aromatic includes dispensing the aromatic into the condensate to produce an aromatic condensate, and energizing the condensate includes energizing the aromatic condensate to generate an aromatic aerosol including the negative ions.

Clause 46. The method of any of clauses 38 to 45, wherein the dispensing the aromatic further includes energizing the aromatic at an aromatic aerosol generator to generate an aromatic aerosol that is combined with the conditioned air.

Clause 47. The method of clause 46, further comprising controlling the aromatic aerosol generator to generate the aromatic aerosol only when the aerosol including the negative ions is generated.

Clause 48. An air purifying system comprising: a heating, ventilation and air conditioning (HVAC) system with HVAC equipment configured to provide conditioned air to a conditioned space, the HVAC system including a condensate trap configured to collect condensate produced during operation; an air purifying device configured to energize the condensate from the condensate trap to generate an aerosol including negative ions; and a fan operable to move the aerosol toward the conditioned space to which the conditioned air is provided by the HVAC system, the aerosol being combinable with the conditioned air provided by the HVAC system.

Clause 49. The air purifying system of clause 48, wherein the HVAC system is set up in an installation with a duct that defines a pathway to a location proximate an ingress to the conditioned space, and the fan is operable to move the aerosol through the duct, toward the location and thereby the ingress to the conditioned space.

Clause 50. The air purifying system of clause 48 or clause 49, wherein the air purifying device includes a conduit and an aerosol generator, the HVAC system is set up in an installation with an air curtain located proximate an ingress to the conditioned space, and the air curtain includes the aerosol generator and the fan, and wherein the conduit is configured to deliver the condensate from the condensate trap to the air curtain and thereby the aerosol generator, and the aerosol generator is configured to energize the condensate.

Clause 51. The air purifying system of any of clauses 48 to 50, further comprising a door sensor configured to provide an indication of an open-closed state of a door at an ingress to the conditioned space, and the HVAC system further includes control circuitry operably coupled to the HVAC equipment, the door sensor and the fan, wherein the control circuitry is configured to determine the open-closed state of the door using the door sensor, and control operation of the fan based on the open-close state of the door.

Clause 52. The air purifying system of clause 51, wherein the control circuitry configured to determine the open-closed state of the door includes the control circuitry configured to determine a transition of the door from a closed state to an open state, and the control circuitry configured to control the operation of the fan includes the control circuitry configured to increase an operating speed of the fan in response to the transition.

Clause 53. The air purifying system of clause 51 or clause 52, wherein the control circuitry configured to determine the open-closed state of the door includes the control circuitry configured to determine a transition of the door from a closed state to an open state, and the control circuitry configured to control the operation of the fan includes the control circuitry configured to turn the fan on in response to the transition.

Clause 54. The air purifying system of any of clauses 51 to 53, wherein the open-closed state of the door indicates the door is in a closed state, and the control circuitry configured to control the operation of the fan includes the control circuitry configured to control the fan to continuously operate when the door is in the closed state.

Clause 55. The air purifying system of any of clauses 48 to 54, wherein the HVAC system is configured to provide the conditioned air to content within the conditioned space, and the HVAC system further includes control circuitry operably coupled to the HVAC equipment and the air purifying device, the control circuitry configured to: determine a type of the content within the conditioned space; and control a rate at which the aerosol is generated at the air purifying device based on the type of the content.

Clause 56. The air purifying system of clause 55, wherein the type of the content is a plant or a perishable food.

Clause 57. The air purifying system of any of clauses 48 to 56, wherein the conditioned space is divided into compartments to which the conditioned air is provided, and the fan is operable to move portions of the aerosol toward respective ones of the compartments.

Clause 58. The air purifying system of any of clauses 48 to 57, wherein air purifying system comprises air purifying devices that include the air purifying device, and fans that include the fan, wherein the air purifying devices include conduits and aerosol generators, the conditioned space is divided into compartments to which the conditioned air is provided, and the HVAC system is set up in an installation with the aerosol generators and the fans for respective ones of the compartments, and wherein the conduits are configured to deliver the condensate from the condensate trap to the aerosol generators at which the condensate is energized to generate respective aerosols including negative ions, and the fans are operable to move the respective aerosols toward respective ones of the compartments.

Clause 59. The air purifying system of any of clauses 48 to 58, wherein the HVAC equipment includes conditioning coils, the aerosol includes droplets of the condensate, and the fan is operable to direct the aerosol over the conditioning coils on which those of the droplets that contact the coils freeze and thereby form a layer of frost on the coils.

Clause 60. The air purifying system of any of clauses 48 to 59, wherein the HVAC equipment includes a transport refrigeration unit configured to provide the conditioned air to the conditioned space.

Clause 61. A purifying system comprising: a heating, ventilation, and air conditioning (HVAC) system with HVAC equipment configured to provide conditioned air, the HVAC system including a condensate trap configured to collect the condensate; a purifying device configured to energize the condensate from the condensate trap to generate an ionized fluid; and one or more ice storage containers configured to store the ionized fluid, the one or more ice storage containers thermally coupled to a refrigerant circuit usable to remove heat from the ionized fluid within the one or more ice storage containers, and thereby freeze the ionized fluid to form an ionized solid that is usable as a disinfectant.

Clause 62. The purifying system of clause 61, further comprising a receptacle configured to store the ionized solid with a perishable food to preserve the perishable food.

Clause 63. The purifying system of clause 61 or clause 62, wherein the refrigeration circuit includes a heat exchanger, an ice storage tank within which the one or more ice storage containers are loaded, and from which the one or more ice storage containers are unloadable, and wherein the refrigeration circuit is configured to circulate a refrigerant fluid to transfer thermal energy between the one or more ice storage containers loaded within the ice storage tank and the heat exchanger.

Clause 64. The purifying system of any of clauses 61 to 63, further comprising: a particle filter configured to filter the condensate as the condensate is collected by the condensate trap; and an ultraviolet light source configured to disinfect the condensate in the condensate trap, and wherein the ionized fluid is generated from the condensate as filtered and disinfected.

Clause 65. The purifying system of any of clauses 61 to 64, wherein the HVAC equipment includes a transport refrigeration unit that includes the refrigeration circuit with a heat exchanger at which the condensate is produced and routed to the purifying device.

Clause 66. A method of disinfecting a conditioned space to which a heating, ventilation, and air conditioning (HVAC) system with HVAC equipment is configured to provide conditioned air, the HVAC system including a condensate trap configured to collect condensate produced during operation, the method comprising: delivering the condensate from the condensate trap to an aerosol generator; energizing the condensate at the aerosol generator to generate an aerosol including negative ions; and operating a fan to move the aerosol toward the conditioned space to which the conditioned air is provided by the HVAC system, the aerosol combined with the conditioned air provided by the HVAC system.

Clause 67. The method of clause 66, wherein the HVAC system is set up in an installation with a duct that defines a pathway to a location proximate an ingress to the conditioned space, and the fan is operated to move the aerosol through the duct using a fan, toward the location and thereby the ingress to the conditioned space.

Clause 68. The method of clause 66 or clause 67, wherein the HVAC system is set up in an installation with an air curtain located proximate an ingress to the conditioned space, and the air curtain includes the aerosol generator and the fan, and wherein the condensate is delivered from the condensate trap to the air curtain and thereby the aerosol generator.

Clause 69. The method of any of clauses 66 to 68, wherein the conditioned space further includes a door at an ingress to the conditioned space, and the method further comprises: determining an open-closed state of the door using a door sensor; controlling operation of the fan based on the open-close state of the door.

Clause 70. The method of clause 69, wherein determining the open-closed state of the door includes determining a transition of the door from a closed state to an open state, and controlling the operation of the fan includes increasing an operating speed of the fan responsive to the transition.

Clause 71. The method of clause 69 or clause 70, wherein determining the open-closed state of the door includes determining a transition of the door from a closed state to an open state, and controlling the operation of the fan includes turning the fan on responsive to the transition.

Clause 72. The method of any of clauses 69 to 71, wherein the open-closed state of the door indicates the door is in a closed state, and controlling the operation of the fan includes controlling the fan to continuously operate when the door is in the closed state.

Clause 73. The method of any of clauses 66 to 72, wherein the HVAC system is configured to provide the conditioned air to content within the conditioned space, and the method further comprises: determining a type of the content within the conditioned space; and controlling a rate at which the aerosol is generated at the aerosol generator based on the type of the content.

Clause 74. The method of clause 73, wherein the type of the content is determined as a plant or a perishable food.

Clause 75. The method of any of clauses 66 to 74, wherein the conditioned space is divided into compartments to which the conditioned air is provided, and the fan is operated to move portions of the aerosol toward respective ones of the compartments.

Clause 76. The method of any of clauses 66 to 75, wherein the conditioned space is divided into compartments to which the conditioned air is provided, and the HVAC system is set up in an installation with aerosol generators and fans for respective ones of the compartments, and wherein the condensate is delivered from the condensate trap to the aerosol generators at which the condensate is energized to generate respective aerosols including negative ions, and the fans are operated to move the respective aerosols toward respective ones of the compartments.

Clause 77. The method of any of clauses 66 to 76, wherein the HVAC equipment includes conditioning coils, the aerosol includes droplets of the condensate, and the method further comprises directing the aerosol over the conditioning coils on which those of the droplets that contact the coils freeze and thereby form a layer of frost on the coils.

Clause 78. The method of any of clauses 66 to 77, wherein the HVAC equipment includes a transport refrigeration unit configured to provide the conditioned air to the conditioned space.

Clause 79. A method of forming a disinfectant from condensate produced by and during operation of a heating, ventilation, and air conditioning (HVAC) system with HVAC equipment configured to provide conditioned air, the HVAC system including a condensate trap configured to collect the condensate, the method comprising: delivering the condensate from the condensate trap to an aerosol generator; energizing the condensate at the aerosol generator to generate an ionized fluid including negative ions; and freezing the ionized fluid to form an ionized solid that is usable as a disinfectant.

Clause 80. The method of clause 79, wherein the method further comprises storing the ionized solid with a perishable food to preserve the perishable food.

Clause 81. The method of clause 79 or clause 80, wherein the method further comprises receiving the ionized fluid within one or more ice storage containers, the one or more ice storage containers being thermally coupled to a refrigeration circuit, and wherein the ionized fluid is frozen using the refrigeration circuit to remove heat from the ionized fluid within the one or more ice storage containers.

Clause 82. The method of clause 81, wherein the refrigeration circuit includes a heat exchanger, and an ice storage tank within which the one or more ice storage containers are loaded, and from which the one or more ice storage containers are unloadable, and wherein freezing the ionized fluid includes circulating a refrigerant fluid within the refrigeration circuit to transfer thermal energy between the one or more ice storage containers loaded within the ice storage tank and the heat exchanger.

Clause 83. The method of any of clauses 79 to 82, further comprising: filtering the condensate as the condensate is collected by the condensate trap; and disinfecting the condensate in the condensate trap using an ultraviolet light source, and wherein the ionized fluid is generated from the condensate as filtered and disinfected.

Clause 84. The method of any of clauses 79 to 83, wherein the HVAC equipment includes a transport refrigeration unit that includes the refrigeration circuit with a heat exchanger at which the condensate is produced and routed to the aerosol generator.

A method according to any of clauses 27-47 or any of clauses 66-78 may be conducted using an air purifying device in accordance with clause 21-26, an air purifying system according to any of clauses 1-20 or 48-60, a purifying system according to any of clauses 61-65. An air purifying system according to any of of clauses 1-20 or 48-60, or a purifying system according to any of clauses 61-65 may comprise an air purifying device in accordance with any of clauses 21-26. The HVAC system of a method in accordance with any of clauses 79-84 may form part of or be associated with an air purifying system according to any of of clauses 1-20 or 48-60, or a purifying system according to any of clauses 61-65, or an air purifying device in accordance with any of clauses 21-26.

Many modifications and other implementations of the disclosure set forth herein will come to mind to one skilled in the art to which the disclosure pertains having the benefit of the teachings presented in the foregoing description and the associated figures. Therefore, it is to be understood that the disclosure is not to be limited to the specific implementations disclosed and that modifications and other implementations are intended to be included within the scope of the appended claims. Moreover, although the foregoing description and the associated figures describe example implementations in the context of certain example combinations of elements and/or functions, it should be appreciated that different combinations of elements and/or functions may be provided by alternative implementations without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. An air purifying device for a heating, ventilation and air conditioning (HVAC) system operable to provide conditioned air to a conditioned space, the HVAC system including a condensate trap configured to collect condensate produced during operation of the HVAC system, the air purifying device comprising:
an aerosol generator; and
a conduit operably coupleable to and between the condensate trap and the aerosol generator, the conduit configured to deliver the condensate from the condensate trap to the aerosol generator, the aerosol generator configured to energize the condensate to generate an aerosol including negative ions combinable with the conditioned air provided by the HVAC system for delivery to the conditioned space.

2. The air purifying device of claim 1, wherein the aerosol generator comprises:
at least one piezo transducer; and
a driver circuit configured to drive the at least one piezo transducer to vibrate and break up the condensate into the aerosol that includes droplets and the negative ions.

3. The air purifying device of claim 2, wherein the aerosol generator is configured such that the droplets of the aerosol are liquid nanoparticles.

4. The air purifying device of claim 2 or 3, wherein the at least one piezo transducer is two or more piezo transducers, and the driver circuit is configured to drive the two or more piezo transducers to synchronously vibrate.

5. The air purifying device of any preceding claim, wherein the aerosol generator is configured to generate the aerosol with the negative ions under control from the HVAC system, and based on an indication of occupancy in the conditioned space.

6. An air purifying system comprising:
a heating, ventilation and air conditioning (HVAC) system with HVAC equipment configured to provide conditioned air to a conditioned space, the HVAC system including a condensate trap configured to collect condensate produced during operation; and
an air purifying device configured to energize the condensate from the condensate trap to generate an aerosol including negative ions, the aerosol being combinable with the conditioned air provided by the HVAC system for delivery to the conditioned space.

7. The air purifying system of claim 6, wherein:
the HVAC system is set up in an installation that defines an air supply path, and
the air purifying device includes an aerosol generator located in the air supply path and configured to energize the condensate;
optionally wherein:
the HVAC equipment includes a heat exchanger coil located in the air supply path, and
wherein the heat exchanger coil is arranged downstream from the aerosol generator in the air supply path.

8. The air purifying system of claim 6 or 7, further comprising:
a particle filter configured to filter the condensate as the condensate is collected by the condensate trap; and
an ultraviolet light source configured to disinfect the condensate in the condensate trap.

9. The air purifying system of any of claims 6 to 8, wherein the HVAC system is configured to provide the conditioned air combined with the aerosol including the negative air ions, to the conditioned space and an outdoor environment.

10. The air purifying system of any of claims 6 to 9, wherein the HVAC system includes control circuitry operably coupled to the HVAC equipment and the air purifying device, and
wherein the HVAC equipment further includes a sensor configured to provide an indication of occupancy in the conditioned space, and the control circuitry is configured to control the air purifying device to generate the aerosol with the negative ions based on the indication of occupancy.

11. The air purifying system of any of claims 6 to 10, wherein the HVAC system includes control circuitry operably coupled to the HVAC equipment and the air purifying device, and the control circuitry is configured to control the air purifying device to generate the aerosol with the negative ions during a first time interval, based on an expected conditioned load for the conditioned space during a second time interval that is after the first time interval.

12. The air purifying system of any of claims 6 to 11, wherein the HVAC system includes control circuitry operably coupled to the HVAC equipment and the air purifying device, and
wherein the HVAC equipment further includes a humidity sensor configured to provide measurements of a humidity level in the conditioned space, and the control circuitry is configured to adjust a rate at which aerosol is generated by the air purifying device based on the measurements of the humidity level.

13. A method of disinfecting a conditioned space to which heating, ventilation, and air conditioning (HVAC) equipment of an HVAC system is configured to provide conditioned air, the HVAC system including a condensate trap configured to collect condensate produced during operation, the method comprising:
delivering the condensate from the condensate trap to an aerosol generator;
energizing the condensate at the aerosol generator to generate an aerosol including negative ions;
combining the aerosol with the conditioned air provided by the HVAC system; and
delivering the combined air and aerosol to the conditioned space.

14. The method of claim 13, wherein energizing the condensate includes driving at least one piezo transducer to vibrate and break up the condensate into the aerosol that includes droplets and the negative ions;
optionally wherein the aerosol is generated such that the droplets are liquid nanoparticle droplets and/or liquid microparticles.

15. The method of claim 13 or 14, further comprising dispensing an aromatic that is also combined with the conditioned air provided by the HVAC system, and
wherein delivering the combined air and aerosol includes delivering the combined conditioned air, aerosol, and aromatic to the conditioned space.
